Europäisches Patentamt

European Patent Office

Office européen des brevets

(19)

(11) Veröffentlichungsnummer: **0 209 790**
**B1**

## (12) EUROPÄISCHE PATENTSCHRIFT

(45) Veröffentlichungstag der Patentschrift: **19.09.90**

(21) Anmeldenummer: **86109281.5**

(22) Anmeldetag: **08.07.86**

(51) Int. Cl.⁵: **C 02 F 3/10,** C 02 F 1/28,
A 01 G 31/00, C 12 N 11/04

(54) **Verfahren zur Herstellung von Füllstoffe enthaltenden, Polymer-gebundenen Trägermassen, die nach diesem Verfahren erhaltenen Trägermassen und ihre Verwendung.**

(30) Priorität: **23.07.85 DE 3526184**

(43) Veröffentlichungstag der Anmeldung:
**28.01.87 Patentblatt 87/05**

(45) Bekanntmachung des Hinweises auf die
Patenterteilung:
**19.09.90 Patentblatt 90/38**

(84) Benannte Vertragsstaaten:
**AT BE CH DE FR GB IT LI NL SE**

(56) Entgegenhaltungen:
**EP-A-0 121 851**
**EP-A-0 150 747**
**DE-A-2 805 607**

**PATENT ABSTRACTS OF JAPAN, Band 7, Nr.**
**167 (C-177)1312r, 22. Juli 1983, Seite 163 C 177;**
**& JP-A-58 76 435 & JP-A56 173 799**

(73) Patentinhaber: **BAYER AG**
**D-5090 Leverkusen 1 Bayerwerk (DE)**

(72) Erfinder: **Reischl, Artur, Dr.**
**H.T.-von-Böttinger-Strasse 19**
**D-5090 Leverkusen 1 (DE)**
Erfinder: **Mack, Kurt, Dr.**
**Claudiusweg 15**
**D-5600 Wuppertal 1 (DE)**

EP 0 209 790 B1

**Beschreibung**

Die Erfindung betrifft eine neues Verfahren zur Herstellung von Füllstoffe enthaltenden, polymergebundenen Trägermassen durch Vermischen der Füllstoffe mit einem wäßrigen Bindemittel auf Polymer-Basis unter anschließender Koagulation des in Wasser dispergierten Polymeren, die nach dem Verfahren erhaltenen Trägermassen und ihre Verwendung bei der biologischen Abwasserreinigung, als Träger bei der biologischen Fermentation in Biokonversionsprozessen, als Träger für Pflanzenwachstum oder als Adsorbentien für feindispergierte Stoffe, insbesondere Rohöl.

Es wurden schon eine Reihe von Verfahren zur Imprägnierung von Schaumstoffen und Schaumstoffteilchen beschrieben, indem man die Schaumstoffe mit einer Reaktivkomponente, z.B. mit Polyisocyanaten, tränkt und nachträglich der Umsetzung mit der anderen Reaktionskomponente, z.B. Polyolen, Polyaminen oder Dämpfen von Diaminen aussetzt, beispielsweise nach Verfahren der DE—OS 3 039 146, Ja 50—103 571, FR—PS 1 587 855, FR—PS 1 574 789, DE—OS 2 131 206 oder US—PS 2 955 056.

Man kann auch die Schaumstoffe einer quellend wirkenden Flüssigkeit aussetzen und bringt danach die Polyurethan-Reaktionskomponenten zur Einwirkung, wodurch Verhärtungen und Versteifungen des Schaumstoffes möglich sind und gegebenenfalls Einlagerungen in der gequollenen Schaumstoff-Matrix, z.B. nach Verfahren der FR—PS 1 341 717, 1 587 855, 1 574 789 oder der DE—AS 1 911 645, erfolgen. Solche Matrix-Schaumstoffe weisen weiterhin typische Schaumstoffeigenschaften, wenn auch andere Härte, Elastizität oder chemische bzw. mechanische Eigenschaften auf.

Eine Vielzahl weiterer Patentschriften beschreibt die Verklebung bzw. die Verpressung von Schaumstoffteilchen (vorzugsweise Polyurethanweichschaumstoffabfallteilchen) mittels Polyisocyanaten, NCO-Prepolymeren und Polyolen, Polyaminen, Wasser oder anderen Reaktionspartnern, gegebenenfalls unter Zusatz von Kork, Fasern, Zellulosepulver, Flammschutzmitteln, Pigmenten, Metallpulvern oder Ruß zu neuartigen Verbundmaterialien, welche gegebenenfalls mit Überzügen oder mit Folien oder Metallplatten versehen oder damit verschweißt werden können. Derartige Verbundmaterialien werden beispielsweise als Isolier- und Dämmplatten, Auskleidungen, Matratzen oder Formkörper verwendet. Entsprechende Verfahren werden beispielsweise in den DE—OS 2 940 260, GB—PS 1 337 413, DE—OS 3 213 610, GB—PS 1 540 076, US—PS 4 254 177, DE—OS 2 908 161, DE—OS 3 120 121 und JA 57/028 180 beschreiben.

Technische Bedeutung hat bischer jedoch praktisch nur die Herstellung von Verbundblockschaum aus zerkleinertem Polyurethanschaumstoff, 10—20 Gew.-% Isocyanatverbindungen, bis etwa 10 Gew.-% Füllstoffen und geringen Mengen Wasser erlangt. Der Füllstoff besteht dabei hauptsächlich aus Farbpigmenten, um den Verbundschaum, der aus verschiedenfarbenen Schaumstoffchargen bestehen kann, eine einheitliche Färbung zu verleihen. Das bei der Herstellung des Verbundschaumstoffes verwendete Wasser bewirkt als Reaktionskomponente die Überführung der Polyisocyanatgruppen in Polyharnstoffgruppen unter Kohlendioxidentwicklung. Die Menge Wasser wird dabei so gewählt, daß sie etwa dem stöchiometrischen Bedarf der Isocyanate entspricht, höchstens aber nur in einem relativ kleinen Überschuß vorhanden ist, da sonst die Entfernung der Feuchtigkeit aus denn 40—60 cm dicken Verbundblöcken Schwierigkeiten bereitet.

Erfindungsgemäß sollen jedoch hoch wasseraufnahmefähige, vorgefertigte Schaumstoffe in stückiger Form und/oder fossile Lignozellulosen und/oder Kohlepulver und gegebenenfalls weitere Füllstoffe enthaltende, hochgefüllte, gegebenenfalls ionisch modifizierte Polymermassen hergestellt werden, welche in Wasser relativ stark gequollen sind und/oder welche in bzw. zwischen den Schaumstoff- und/oder den Füllstoff- bzw. in den Füllstoffteilchen hohe Mengen an Wasser binden, so daß die Wasseraufnahmefähigkeitswerte (WAF) mindestens 30 Gew.-%, vorzugsweise $\geq$50 Gew.-% und bis 97 Gew.-%, (siehe Meßmethode) betragen , und welche insbesondere als Trägermassen für die biologische Abwasserreinigung verwendet werden können.

Bei der biologischen Abwasserreinigung wurden bereits viele Verfahren vorgeschlagen, um den Abbaueffekt zu steigern und um ein möglichst schadstofffreies, gereinigtes Wasser zu erhalten.

So wurde eine Oxidation der Schadstoffe unter erhöhter Sauerstoffzufur zum Belebtschlamm erprobt, weiterhin spezielle Oxidationsverfahren, wie beispielsweise eine Ozon- oder Wasserstoffperoxid-Behandlung.

Es wurde die katalytische Oxidation der Abwasserinhaltsstoffe mittelt Luft und unter Zugabe von Aktivkohle in Verbindung mit einer nachgeschalteten Fällung empfohlen (siehe z.B. DE—PS 22 39 406, DE—OS 30 25 353, A. Bauer et al., Chemie-Technik, Heft 6, Seite 3—9 (1982), K. Fischer et al., GWF-Wasser/Abwasser, Heft 2, Seite 58—64 (1981); R. E. Perrotti et al., Chemical Engineering Progress (CEP), Vol. 69 (11), 63—64 (1973), G. Wysocki et al., ZC-Chemie-Technik, 3 (6), 205—208 (1974) und 3. Bericht "Adsorptive Abwasserreinigung" (Oktober 1975) des Ausschusses Wasser und abwasser beim VCleV.).

Die vorgenannten Verfahren erwiesen sich jedoch technisch als zu aufwendig oder zu kostspielig oder der Abbaueffekt ist noch ungenügend. Die zahlreichen Versuche, Aktivkohole in der Wasserklärtechnik zu verwenden, scheiterten bisher trotz verbesserter Abbauleistung, weil die Aktivkohle sogar in ihrer gebundenen (granulierten) Form bereits bei sehr geringen Strömungen, die wenigstens zeitweise in den Klärbecken zwingend erforderlich sind, in einer nicht tolerierbaren Menge zerrieben und ausgetragen werden. Erfolgreiche Versuche ener ausreichend wirksamen hohen Menge und starken Bindung der Aktivkohle bei gleichzeitiger Erhaltung der Bioaktivität im Klärbecken sind bisher nicht bekannt geworden.

In der DE—OS 30 32 882 (EP—A 46 900) und DE—OS 30 32 869 (EP—A 46 901) wird die Verwendung

2

eines makroporösen Stoffes mit geringem spezifischem Gewicht (10—200 kg/m³) als Trägermaterial für nitrifizierende Bakterien bei der Belebtschlammklärung beschrieben. Es handelt sich z.B. um typische Polyurethanschaumstoffe. In ähnlicher Weise werden solche Schaumstoffteilchen in einem Verfahren und einer Vorrichtung zur anaeroben biologischen Abwasserreinigung beschrieben, Zu den bereits hier erzielten verbesserten Ergebnissen siehe z.B. gwf-Wasser/Abwasser, 124, (1983), Heft 5, 233—239. Derartige Schaumstoffe schwimmen jedoch in freien Belebschlammbecken auf und führen zu verschiedenen Störungen des Prozesses. Stückige Schaumstoffe auf (u.a.) Polyurethanbasis wurden auch in verschiedenen, speziellen Verfahren als Schüttkörperfüllung (DE—PS 28 39 872 und DE—AS 2 550 818) oder als Tropfkörpermasse (österreichische PS 248 354) für die biologische Abwasserreinigung empfohlen.

Die Verwendung von relativ abriebfesten Polyurethanharnstoffschaumstoffen mit offener Zellstruktur mit einem Harnstoff-Urethanverhältnis von (5 wird in der US—PS 45 03 150 als Trägermedium für mikrobiologisch aktive Zellen beim Abwasserreinigungsverfahren beschrieben. Dort werden zum Stand der Technik eine Reihe weiterer Veröffentlichungen angeführt, welche die Verwendung von Schaumstoffen bei der biologischen Abwasserreinigung beschreiben.

In der EP—A—77 411 wird die Verwendung von PUR-Schaumstoffstücken als Filtriermedium beschrieben, dessen Schmutzbeladung nach bestimmten Spülverfahren von Zeit zu Zeit zur Regenerierung des Schaumstoffs abgespült wird.

Auch die Kombination von oberflächenaktiven Feststoffen mit Mikroorganismen zur Erhöhung deren Aktivität bei Biokonversionsprozessen ist bekannt, beispielsweise wird in den DE—OS 26 33 259 und DE—OS 27 03 834 die Adsorption von Zellen an z.B. Aluminiumoxid, Bentonite und $SiO_2$ und deren nachfolgende Einbettung in Polyacrylate beschrieben. Ferner beschreibt die DE—OS 26 29 692 die Einlagerung von Zellen in photohärtbare Polyurethane, die photohärtbare Acrylat-Doppelbindungen enthalten.

Ebenso ist die Einbettung von wachstumsfähigen Zellen in Polyurethan-Hydrogelen bekannt: siehe z.B. Tanaka et al., European Journal of Applied Microbiology and Biotechnology, 7, (1979) ab Seite 371. Auch in der DE—OS 29 29 872 wurde ein Verfahren zur Herstellung von hydrophilen, gelförmigen oder geschäumten Biokatalysatoren mit hoher Beladung an enzymatisch aktiver Substanz durch Polymereinschluß ganzer Zellen von Zellfragmenten oder Enzymen, durch Mischen einer wäßrigen Suspension der enzymatisch aktiven Substanz mit hydrophilen Polyisocyanaten unter Bildung eines enzymatisch hochaktiven, hydrophilen Polyurethan-Netzwerks in Blockform oder perlförmiger Form beschrieben. Auf Seite 7 der genannten Offenlegungsschrift werden zum Stand der Technik auch weitere Veröffentlichungen genannt. Ebenso wurde durch J. Klein und M. Klug in Biotechnology Letters, Vol. 3, No. 2, Seite 65—90 (1981) die Immobilisierung von "microbial cells" in PUR-Matrices wie PUR-Schaumstoffen oder PUR-Gelen beschrieben. Aus EP—A 121 851 ist es ebenfalls bekannt, biologisch aktive Zusammensetzungen auf Basis von oberflächenaktiver Kohle, Polymere mit kationischen Gruppen und wachstumsfähige Zellen enthaltender Hydrogele herzustellen. Dabei erfolgt eine Umsetzung von Isocyanat-Prepolymeren mit wäßrigen Suspensionen, die oberflächenaktive Kohle und Polymere mit kationischen Gruppen enthalten, wobei die wachstumsfähigen Zellen zusammen mit der wäßrigen Suspension direkt immobilisiert oder in einem zweiten Schritt dem zunächst erhaltenen Hydrogel zugefügt werden.

Die Herstellung von enzymatisch aktiven Substanzen enthaltenden Polyurethanen ist jedoch schwierig und schließt den Nachteil ein, daß durch die hohe Reaktionsfähigkeit der Isocyanatgruppen eine — zumindest teilweise — Abtötung von Bakterien, Zellen oder eine Inaktivierung von enzymatisch aktivem Material eintritt. In den Beispielen werden z.B. Restaktivitäten von 7 bis 48% bestimmt. So ist es auch nicht günstig, lebende Bakterien in hydrophile Polyurethane bei der Herstellung einzubauen, um sie z.B. für die Reinigung von Abwässern einzusetzen. Die Einbaumenge solcher Bakterien ist begrenzt, ein Großteil der Bakterien wird zudem durch Isocyanatreaktionen desaktiviert und die ständige Herstellung aktiver, bakterienhaltiger Polyurethanmassen und ihre "Lebendlagerung" beinhaltet Herstellungs- und Lagerungsprobleme, um die zumeist mehrere tausend Kubikmeter fassenden Klärbecken mit der erforderlichen Menge und Konzentration an in Polymerer eingebauten Bakterien zu vorsorgen. Eine drastische Minderung der Wachstumsfähigkeit der Bakterien würde selbst beim örtlich direkten Einbau in der Kläranlage wegen ihrer kurzen Überlebungszeit bei der Immobilisierung im Reaktionsmedium eintreten.

Deshalb mußte nach wie vor die Augabe gelöst werden, für neue, wirtschaftliche und wirksame Verfahren zur verbesserten Abwasserreinigung geeignete Verfahren zur Herstellung neuartiger Trägermaterialien zu entwickeln.

Aufgabe der Erfindung ist somit auch die Bereitstellung von hoch wasseraufnahmefähigen, in Wasser nicht aufschwimmenden, hochgefüllten Polymerträgermassen welche bei der biologischen Abwasserreinigung als Träger für Biomassen dienen können.

Diese Aufgabe konnte durch die Bereitstellung des nachstehend näher beschriebenen erfindungsgemäßen Verfahrens gelöst werden.

Gegenstand der Erfindung ist ein Verfahren zur Herstellung von Füllstoffe enthaltenden, Polymergebundenen Trägermassen, dadurch gekennzeichnet, daß man

A) Füllstoffe auf Basis von vorgefertigten, stückigen oder pulverförmigen Schaumstoffen und/oder von fossilen Lignozellulosepulvern und/oder von Kohlenpulvern, sowie gegebenenfalls weiteren anorganischen oder organischen Füllstoffen und/oder gegebenenfalls von lebendem und/oder totem biologischem Zellmaterial in einer Menge, bezogen auf A) und B) von 5 bis 97 Gew.-%,
mit

B) nichtionisch-hydrophilen, anionischen oder kationischen wäßrigen, koagulierbaren und filmbildenden Polymerdispersionen mit einem Feststoffgehalt von 3 bis 60 Gew.-%, gegebenenfalls unter Zusatz von

C) Wasser, so daß der Gesamtwassergehalt, bezogen auf alle Bestandteile, 20 bis 90 Gew.-% beträgt, und gegebenenfalls unter Zusatz von

D) weiteren Hilfs- und Zusatzmitteln

vermischt und anschließend die Polymerdispersion zur Koagulation bringt.

Gegenstand der Erfindung sind auch die nach diesem Verfahren erhaltenen, Füllstoffe enthaltenden, Polymer-gebundenen Trägermassen.

Gegenstand der Erfindung ist schließlich auch die Verwendung der nach diesem Verfahren erhaltenen, Füllstoffe enthaltenden, Polymer-gebundenen Trägermassen als Träger für inkorporierte oder aufwachsende Biomassen bei der Abwasserreinigung, insbesondere bei der biologischen Abwasserreinigung, als Träger bei der biologischen Fermentation in Biokonversionsprozessen, als Träger für Pflanzenwachstum oder als Adsorbentien für feindispergierte Stoffe und/oder Rohöl.

Die Füllstoffe A sind wesentlicher Bestandteil der erfindungsgemäßen, wasseraufnahmefähigen Polymerträgermassen, wobei durch eine Wechselwirkung zwischen den Füllstoffen und der Polymerträgermasse die unerwartet hohe Wasseraufnahmefähigkeit und auch der hohe Abbaueffekt bei der erfindungsgemäßen Verwendung der erfindungsgemäßen Trägermassen eintritt. Entweder durch die Polymeren und/ oder (bevorzugt) durch hydrophile oder zellige Füllstoffe (z.B. Braunkohle oder Schwarztorf und/oder durch die PUR-Schaumstoffe) wird eine solche Hydrophilie, d.h. Wasseraufnahmefähigkeit in die Träger eingebracht, daß sie den genannten Wasseraufnahmefähigkeitswerten entsprechen.

Geeignete Füllstoffe A) sind z.B. zellige Kunststoffe.

Beispiele hierfür sind Polymerisate oder Copolymerisate von Ethylen, Styrol, Acrylnitril, (Methyl)-Butadien und anderen Vinylverbindungen. Geeignet sind somit beispielsweise geblähte Polystyrolgranulate, expandiertes Polyethylen oder Schaumstoffabfälle auf Polymerisatbasis. Diese Füllstoffe sind jedoch weniger bevorzugt und werden allenfalls als Mischkomponenten neben den bevorzugten Füllstoffen A) verwendet. Zu den bevorzugten Füllstoffen A) gehören beispielsweise vorgefertigte Polyurethan-Schaumstoffteilchen oder Polyurethan(halb)hartschaumstoff-Granulate oder Pulver.

Ökonomisch besonders interessant ist die Verwendung von zelligen Kunststoffen, insbesondere von den in riesigen Mengen zwangsweise anfallenden PUR-Weichschaumstoffabfällen. Diese PUR-Schaumstoffabfälle, vorzugsweise auf Polyetherbasis, können in Form eines preisgünstig erhältlichen, unregelmäßigen, stückigen Granulats mit Kantenlängen von einigen mm bis zu mehreren cm, auch als Mischung verschiedener Schaumstoff-Raumgewichte, als Füllstoff unter Bildung mit den anionischen Polyurethanharnstoffmassen zur Herstellung der Träger verwendet werden. Bevorzugt werden PUR-Schaumstoffteilchen mit Raumgewichten von etwa 10 bis 110 kg/m$^3$ in die PU-Matrix eingebaut. Insbesondere werden die PUR-Weichschaumstoffe in stückiger Form verwendet, während harte, spröde Polyurethanteilchen vorzugsweise in pulverisierter Form eingesetzt werden. Überraschenderweise sind jedoch selbst Abfallweichschaumstoff-Flocken mit einer mittleren Dichte von unter 50 kg/m$^3$ in erfindungsgemäß gebundener Form hervorragend geeignete Träger für aufwachsende Biomassen. Die Hohlräume der Schaumstoffe werden bei der Bindung mit der Polymer-Matrix praktisch ganz oder zumindest teilweise gefüllt und somit steigt das Raumgewicht und die mechanische Festigkeit hinreichend an, damit die Schaumstoff-Flocken nicht mehr aufschwimmen und auch gegenüber mechanischen Einflüssen in Wasser dauerhaft resistent sind.

Weitere Füllstoff A), die allein ode gegebenenfalls zusammen mit den Schaumstoff-Füllstoffen eingesetzt werden können, sind z.B. fossile Lignozellulosen oder deren Folgeprodukte enthaltende Naturstoffe wie insbesondere Braunkohle. Sie ergeben gleichfalls in Folge ihrer hohen Wasserbindekraft hoch wasseraufnahmefähige Träger. Die Braunkohle stellt einen ganz besonders vorteilhaften, hydrophil wirkenden Füllstoff dar und ist besonders bevorzugt, wobei die Braunkohle vorzugsweise als alleiniger Füllstoff oder in Kombination mit Polyurethanschaumstoffteilchen und gegebenenfalls weiteren Füllstoffen Verwendung findet.

Braunkohle vermag hohe Mengen an Wasser hydrophil zu binden, ohne das sich diese Materialien naß anfühlen, so lassen sich z.B. mehr als 150% Wasser, bezogen auf Braunkohle-Trockensubstanz binden. Außerdem ergibt Braunkohle günstige topologische Bedingungen für die Herstellung von porösen Trägermassen, wie sie sich als Träger bei der biologischen Abwasserreinigung besonders eignen.

Braunkohle aus fossilen Lagerstätten, beispielsweise aus dem Aachener Braunkohlenrevier weist im allgemeinen einen Wassergehalt von ca. 60 Gew.-% auf. Diese wasserhaltige Braunkohle kann im Falle der Verwendung von kationischen Polymerdispersionen als Komponente B) als solche verwendet werden; lediglich bei Verwendung von anionischen Polymerdispersionen ist oftmals eine Reduzierung des Wassergehalts der Braunkohle vor ihrer Verwendung als Füllstoff empfehlenswert, da mit dieser Trocknung eine oftmals erwünschte Reduzierung des Gehalts der Kohle an wasserlöslichen Bestandteilen, die sich bei der Verwendung von anionischen Polymerdispersionen störend auswirken, einhergeht.

Dies kann am einfachsten dadurch erreicht werrden, daß man die wasserhaltige Braunkohle einem Trocknungsprozeß unterwirft und den Wassergehalt stark herabsetzt, mindestens unter 20 Gew.-%, besser

4

unter 10 Gew.-% Mit fortschreitender Abnahme des natürlichen Feuchtigkeitsgehalts und Zunahme der Trocknungstemperatur und -dauer erfolgen in einem Temperungsprozeß Umwandlungs- bzw. Kondensationsreaktionen unter Molekülvergroßerung, welche die Wasserlöslichkeit der Huminsäuren stark reduzieren und die Braunfärbung des Wassers, in dem eine Probe an Braunkohle z.B. suspendiert ist, verringert.

Nach diesem Trocknungsprozeß ist die Braunkohle als Füllstoff für die bevorzugte, erfindungsgemäße Verwendung als Polyurethanharnstoff-Trägermassen im wäßrigen Millieu wesentlich besser geeignet und deshalb besonders bevorzugt.

Eine weitere Methode, die Anteile löslicher Verbindungen in der Braunkohle zu reduzieren, ist eine chemische Behandlung, beispielsweise mit Überschußmengen an Isocyanatverbindungen. Mit Di- und Polyisocyanaten, die monomer oder polymer sein können, reagieren H-acide Gruppen der gegebenenfalls noch mehr oder weniger feuchten Braunkohle ebenfalls unter Molekülvergrößerung und gleichzeitig erfolgt in Anwesenheit von Restfeuchte eine Polyharnstoffumhüllung unter Kohlendioxidabspaltung. Beide Methoden, das Tempern unter Feuchtigkeitsverminderung und/oder -entzug und die Polyisocyanatbehandlung der Braunkohle lassen sich am einfachsten direkt bei der Herstellung der Polymerträgermassen miteinander kombinieren.

Es hat sich jedoch auch gezeigt, daß, mit anionischen Polymeren gebundene Braunkohle enthatende, Träger bei ihrer Verwendung als Trägermassen für Bakterien bei der Abwasserreinigung auch dann noch geeignet sind, wenn zunächst über eine kurze Zeit wasserlösliche Restbestandteile, beispielsweise an Huminsäureverunreinigungen oder gelbfärbenden Verbindungen, ausbluten, weil durch die erfindungsgemäßen Trägermassen die biologische Abwasserreinigung so sehr verbessert wird, insbesondere bei Erhöhung der Schadstoffkonzentration, daß die löslichen Komponenten, soweit sie von der Braunkohle stammen, nicht einmal in der Anlaufperiode nach Einsetzen der Trägermassen im Auslauf der biologischen Reinigungsstufen in Erscheinung treten.

Auch Torf ist im Prinzip als Träger A) erfindungsgemäß geeignet. Jedoch enthält Torf bekanntlich bedeutend höhere Mengen an wasserlöslichen Bestandteilen als Braunkohle, die Wasser sogar dunkelbraun färben. Deshalb ist die Anwendung der oben beschriebenen Maßnahmen zur drastischen Reduzierung der Löslichkeit besonders dringend geboten, insbesondere wenn die erfindungsgemäß bevorzugte Verwendung als Trägermaterial für mikorbielle Umwandlungprozesse vorgesehen ist.

Schwarztorf ist gegenüber Weißtorf grundsätzlich besser geeignet. In einer vorausgehenden Temperung wird dem bis 80 Gew.-% Wasser enthaltenden Schwarztorf (bezogen auf das natürliche Material) der größte Teil des Wassers entzogen, so daß die Restfeuchte möglichst deutlich unter 20 Gew.-%, bevorzugt unter 10 Gew.-%, bezogen auf Gesamtmenge sinkt. Eine Polyharnstoffmodifizierung des weitgehend entwässerten Schwarztorfs mit nieder- oder höhermolekularen Di- oder Polyisocyanaten bei Temperaturen von z.B. 70 bis 110°C bewirkt zusätzlich eine stärkere Reduzierung der wasserlöslichen Anteile. Bevorzugt werden aromatische Di- und Polyisocyanate in einer Menge eingesetzt, daß pro Isocyanatäquivalent (d.s. 42 g Isdocyanatgruppen) 0,5 bis 2,5 kg Schwarztorf, bezogen auf Trockenmasse, umgesetzt werden.

In Fällen störender Anionengruppen wird für das erfindungsgemäße Verfahren der Schwarztorf zur Herstellung der anionischen Polymer-Trägermassen in dieser modifizierten Form bevorzugt eingesetzt. Eine Ausnahme ist jedoch möglich, wenn beispielsweise die Polyurethanharnstoffmassen als Trägermaterial zur Aufnahme von Samen zur Ausbildung von Setzlingen für den Gartenbau Verwendung finden sollen. Hierbei ist das Ausbluten wasserlöslicher Verbindungen aus dem Torf ohne Bedeutung. Hierfür kann sogar der sonst weniger geeignete Weißtorf trotz der gegenüber Schwarztorf höheren Anteile an wasserlöslichen Verbindungen Verwendung finden.

Weitere Füllstoffkomponenten A) sind z.B. andere Kohlen wie Steinkohle, Holzkohle, aktivkohle oder Braunkohlenkoks, die pulverförmig eingesetzt werden und vorzugsweise als Mischkomponente zusammen mit den vorgenannten Schaumstoff- und/oder fossilen Lignozellulosefüllstoffen Verwendung finden. Grundsätzlich ist es jedoch auch möglich, die letztgenannten Füllstoffe als alleinige Komponente A) einzusetzen, wenn dies auch weniger bevorzugt ist und nicht zu der maximalen erfindungsgemäß erzielbaren Wirkung führt.

Weitere geeignete Füllstoffe A) bzw. Füllstoffkomponenten sind beispielsweise Destillationsrückstände der Toluylendiisocyanat-Destillation, welche beispielsweise durch Eintragen der Destillationsrückstände in Wasser unter Denaturierung und anschließender Granulierung erhalten werden. Diese TDI- Rückstände können gegebenenfalls auch noch nachträglich durch Behandlung mit reaktive Wasserstoffe tragende Verbindungen wie Ammoniak, Polyolen oder Polyaminoverbindungen modifiziert sein. In vielen Fällen tragen sie nocht geringe Mengen inkludierter NCO-Gruppen oder reaktive Abwandlungsprodukte von Isocyanaten, welche mit den Biomassen oder abzubauenden Verbindungen reagieren können. Derartige Destillationsrückstände werden beispielsweise in den DE—OS 28 46 814, 28 46 809, 28 46 815 beschrieben.

Auch andere Destillationsrückstände, beispielsweise hochschmelzende Destillationsrückstände, die bei der destillativen Aufarbeitung von Aminen, phenolen oder von Caprolactam anfallen, sind im Prinzip als erfindungsgemäß verwendbare Füllstoffe A) insbesondere als Abmischkomponente mit den bevorzugten Füllstoffen A) geeignet.

Auch anorganische Füllstoffe wie Quarz, Seesand, pyrogene Koeselsäure (Aerosil), Silikate, Alumosilikate, Bentonite, Aluminiumoxid, Bimsstein, Kieselsole, Wasserglas aber auch Calciumoxid,

5

Calciumcarbonat, Schwerspat, Gips, Eisen-II- und/oder Eisen-III-oxide, insbesondere aber auch feinteilige gegebenenfalls magnetische Oxide wie Magnetit, Chrom-(IV)-oxid, Bariumferrite, Eisenpulver oder γ-$Fe_2O_3$ in Pigmentform werden in speziellen Ausführungen in Anteilen gegebenenfalls neben den fossilen Lignozellulosen und/oder Schaumstoff- Füllstoffen und/oder zusammen mit den kohlehaltigen Füllstoffen mitverwendet, um das spezifische Gewicht der Trägermaterialien zu regulieren, so daß diese in der zu klärenden Flüssigkeit absinken oder schweben, jedoch keinesfalls aufschwimmen. Besonders feinteilige anorganische Füllstoffe (z.B. mit Primärteilchen unter 10 μm und hoher Oberfläche, z.B. Aerosil oder Eisenoxide, insbesondere der bei der Eisenoxidherstellung zwangsweise anfallende Magnetit) begünstigen bei Anwesenheit in den erfindungsgemäß zu verwendenden Trägern die Sauerstoffübertragung auf die Klärschlammbakterien und somit bessere Abbauleistungen bzw. Abbauwirkungen, wobei Metalloxide offenbar besonders günstige, spezifische Sauerstoffübertragungsfunktionen bewirken und somit erfindungsgemäß günstige Abbauwirkungen ergeben. Als modifizierende Füllstoffzusätze können auch Fasern (z.B. anorganische Fasern) wie Glasfasern oder natürliche bzw. synthetische Fasern (z.B. Baumwollstaub) mitverwendet werden.

Die mittlere Korngröße der Füllstoffe liegt im allgemeinen zwischen 0,1 bis 1000 μm, bevorzugt unter 300 μm, besonders bevorzugt unter 100 μm, wobei insbesondere für Aktivkohle und anorganische Bestandteile sowie beim Steinkohlenpulver bzw. Holzkohlenpulver geringere Korngrößen bevorzugt werden als etwa im Fall von Torf oder Braunkohlenstaub, wobei Torf und Braunkohle gegebenenfalls faserige Anteile mit mehreren mm Länge enthalten können.

Für als Füllstoffe zu verwendende Schaumstoffteile gilt die Größenbeschränkung der Korngröße nicht. Hierzu können gegebenenfalls einige mm (z.B. 1 bis 40, vorzugsweise 2 bis 20 mm) große Schaumstoffstücke von der Polyurethan(harnstoff)matrix eingebettet werden oder sogar etwa 2 bis 10 mm dicke PUR-Schaumstoff-Folien.

Der gesamte Füllstoffanteil soll in den beim erfindungsgemäßen Verfahren einzusetzenden Gemischen über 5 Gew.-%, vorzugsweise mindestens 30 Gew.-% und besonders bevorzugt mehr als 50 Gew.-%, oftmals mehr als 75 Gew.-% betragen, wobei eine Obergrenze von 97 Gew.-%, vorzugsweise von 95 Gew.-% einzuhalten ist. Diese Prozentangaben beziehen sich auf die Trockensubstanz der Komponenten A) und B). Die unteren Grenzbereiche werden nur in Ausnahmefällen, vor allem bei Verwendung extrem leichter und voluminöser Füllstoffe erreicht. Die Obergrenze wird im allgemeinen von der Kohäsion und der Abriebbeständigkeit der erfindungsgemäßen Trägermassen bestimmt. In Extremfällen ist es sogar möglich, den Füllstoffanteil bis auf 97 Gew.-% zu steigern, wenn man die bei der nachfolgenden erfindungsgemäßen Verwendung der Trägermassen bei der biologischen Abwasserklärung in einer Festbettanordnung arbeitet.

Besonders bevorzugt werden Polymerträgermassen, welche Füllstoffkombinationen von fossilen Lignozellulosen, insbesondere Braunkohlenstaub und/oder Kohlenpulver und/oder PUR-Schaumstoffteilchen (bevorzugt PUR-Weichschaum-Abfallteilchen) enthalten. Günstigste Eigenschaften werden in der Kombination von Polyurethanschaumstoffteilchen mit Braunkohle erhalten. Die neben den genannten, bevorzugten Füllstoffarten zusätzlich zu verwendenden Füllstoffe wie anorganische Füllstoff- oder Destillationsrückstände und andere, bereits erwähnte sonstige Füllstoffe, werden mit Ausnahme der ferromagnetischen Füllstoffe bevorzugt in Mengen <35 Gew.-%, insbesondere <20 Gew.-% verwendet.

Die Mischungsverhältnisse von Schaumstoffteilchen und der bevorzugten Braunkohle können beliebig liegen, jedoch werden bevorzugt Mischungsverhältnisse von 1 zu 10 bis 10 zu 1, besonders bevorzugt 1 zu 5 bis 5 zu 1 eingesetzt.

Die Füllstoffe werden in die Polymermatrix nach verschiedenen, nachstehend noch näher angegebenen Ausführungsformen eingearbeitet.

Der "in situ"-Einbau von Mikroorganismen in Polyurethane oder andere Kunststoffe ist, wie schon erwähnt, selbst unter sehr schonenden und technisch aufwendigen Bedingungen, nicht ohne wesentlichen Verlust an vermehrungsfähigen Bakterien und starker Minderung der Bioaktivität möglich. Die Herstellungsbedingungen sind dabei insbesondere hinsichtlich der Temperatur (etwa +10°C) anzupassen. Trotzdem ist dieses, in speziellen Fällen an sich mögliche Verfahren, nicht bevorzugt und zumeist auch nicht notwendig, da ein Aufwachsen von Biomassen in die Schaumstoff- und Braunkohle- oder Torfhaltigen Polymerträgermassen in hervorragender Weise erfolgt.

Bei den Polymer-Dispersionen B) handelt es sich im Prinzip um beliebige, durch Zugabe eines Koagulanz und/oder durch Hitzeeinwirkung koagulierbare Kunststoffdispersionen, d.h. um wäßrige Dispersionen von durch Polymerisation, Polykondensation oder Polyaddition hergestellten Polymeren, wobei die Dispergierbarkeit der Polymeren in Wasser sowohl durch nichtionische als auch anionische als auch kationische Emulgatoren gewährleistet sein kann, die ihrerseits sowohl in Form von externen, nicht in das Polymergerüst chemisch eingebauten Emulgatoren als auch in Form von chemisch in das Polymergerüst eingebauten internen Emulgatoren vorliegen können. Die wäßrigen Dispersionen weisen im allgemeinen einen Feststoffgehalt von 3 bis 60, vorzugsweise 25 bis 55 Gew.-% auf.

Besonders gut geeignet sind wäßrige Dispersionen von Polymerisaten olefinisch ungesättigter Monomerer oder wäßrige Polyurethandispersionen. Auch natürliche Latices beispielsweise Naturkautschuklatices können verwendet werden. Die geeigneten Polymerisatdispersionen oder Latices weisen im allgemeinen Primärteilchem mit eiem mittleren Teilchendurchmesser von 0,01 bis 10 μm, vorzugsweise von unter 3 μm und insbesondere von unter 1 μm auf. Dies bedeutet, daß die durch Emulsionspolymerisation hergestellten, sehr feinteiligen Latices gegenüber den durch Suspensions- oder Perlpolymerisation hergestellten Polymerisatdispersionen den Vorzug genießen. Allerdings dürfen Agglomerate von Primärteilchen einen mehrfach größeren Teilchendurchmesser aufweisen.

Bezüglich der Herstellung der erfindungsgemäß geeigneten Polymerisatdispersionen sei auf die bekannten Verfahren verwiesen, wie sie beispielsweise in Houben-Weyl, Makromolekulare Stoffe, Band XIV/1 beschrieben sind. Als Ausgangsmaterialien zur Herstellung von erfindungsgemäßen Polymerisatdispersionen eignen sich die üblichen, olefinisch ungesättigten Monomeren, beispielsweise Vinylverbindungen wie Styrol, Acrylnitril, Methacrylnitril, Acrylsäure, Methacrylsäure, (Meth)Acrylsäureester, Allylmethacrylat, Hydroxyalkylacrylat, Acrylsäureamid, Ethylen, Propylen, Vinylchlorid, Vinylacetat, Vinylpyridin oder Halbester der Maleinsäure. Bevorzugte Divinylverbindungen sind Butadien-(1,3), 2-Methylbutadien-(1,3) (Isopren), 2,3-Dimethyl-butadien-(1,3) und 2-Chlor-butadien (Chloropren), p-Divinylbenzol.

Bei den bevorzugten Polymerisat-Dispersionen handelt es sich um elastische Filme bildende Polymerisatdispersionen, während die zu harten und spröden Filmen auftrocknenden Dispersionen im allgemeinen nur als Abmischkomponente Verwendung finden. Von besonderem Interesse sind beispielsweise die durch Emulsionspolymerisation hergestellten Styrol-Butadien-Latices, Latices aus Butadien und Acrylnitril und Methacrylsäure, wobei diese Dispersionen gegebenenfalls durch einpolymerisierte Acryl- oder Methacrylsäurealkylester mit 1 bis 6 Kohlenstoffatomen im Alkylrest modifiziert sein können, die bekannten Polyvinylacetatdispersionen, die durch partielle Verseifung alkoholische gruppierungen aufweisen können, synthetische Polyisoprenlatices oder Naturkautschuklatex. Weitere geeignete Polymerisatdispersionen sind beispielsweise auch die bekannten Polychloropren-Latrices. Besonders bevorzugt sind die entsprechenden kationisch modifizierten Latics, da diese eine fixierende Wirkung für die in Braunkohlenstaub oder Torf vorliegenden löslichen Bestandteile aufweisen. Derartige kationisch modifizierte Latices werden z.B. dann erhalten, wenn als Monomere tert. Stickstoffatome aufweisende Verbindungen mitverwendet werden, die vor, während oder im Anschluß an die Polymerisationsreaktion in kationische Gruppen, beispielsweise durch Neutralisation mit einer geeigneten Säure wie z.B. Salzsäure, Schwefelsäure oder Phosphorsäure, oder durch Quaternierung mit einem Quaternierungsmittel wie beispielsweise Dimethylsulfat überführt werden können. Polymerisatdispersionen mit eingebauten anionischen Zentren können beispielsweise durch Mitverwendung von Carboxylgruppen oder Carboxylatgruppen aufweisenden Monomeren erhalten werden, wobei die Neutralisation der gegebenenfalls vorliegenden Carboxylgruppen mit Basen, wie beispielsweise Natron- oder Kalilauge oder Triethylamin während oder im Anschluß an die Polymerisationsreakition erfolgt. Gegebenenfalls vorliegende externe Emulgatoren sind beispielsweise Fettseifen, Harzseifen, Sulfate oder Sulfonate mit längeren hydrophoben Kohlenwasserstoffresten, Ammoniumsalze auf Basis von Paraffinaminen, Chloride, Sulfate oder Nitrate von Estern auf Paraffinfettsäuren und Aminoalkoholen wie Dimethylaminoethanol oder Polyethylenglykolether von Fettsäuren oder Fettsäureamiden.

Anstelle der beispielhaft genannten Polymerisatdispersionen können alternativ oder in Kombination mit diesen auch wäßrige Polyurethandispersionen Verwendung finden. Es können sowohl nichtionischhydrophil als auch kationisch als auch anionisch modifizierte Polyurethandispersionen Verwendung finden, wobei vorzugsweise solche Polyurethandispersionen verwendet werden, die die jeweiligen "Emulgatoren" in chemisch eingebauter Form enthalten. Die Herstellung von geeigneten wäßrigen Polyurethandispersionen ist bekannt und beispielsweise in "Die Angewandte Makromulekulare Chemie" 98 (1981) auf den Seiten 133—165 oder in "Progress in Organic Coatings" 9 (1981), Seiten 281—340 beschrieben. Kationisch modifizierte Polyether-Polyurethan-Dispersionen werden besonders bevorzugt verwendet, insbesondere, falls fossile Lignozellulosen als Füllstoffe Verwendung finden. Estergruppen aufweisende Polyurethandispersionen sind wegen ihrer Hydrolyseanfälligkeit weniger bevorzugt. Oftmals sind wäßrige Dispersionen von verzweigten Polyurethanen bevorzugt, die als sedimentierende und redispergierbare wäßrige Suspensionen vorliegen.

Die Herstellung der wäßrigen Polyurethandispersionen kann auch so erfolgen, daß man ein gegebenenfalls ionisch modifiziertes NCO-Prepolymer mit Wasser vermischt, wobei dem System gegebenenfalls ein gegebenenfalls ionisch modifiziertes Kettenverlängerungsmittel beispielsweise ein Diamin wie Ethylendiamin, Hexamethylendiamin oder 4,4'-Diaminodicyclohexylmethan (nicht modifiziert) bzw. das Natriumsalz der N-(2-Aminoethyl)-2-aminoethansulfonsäure (anionisch modifiziertes Diamin) einverleibt wird, so daß die Bildung der Polyurethandispersion unter Kettenverlängerung (die auch mit reinem Wasser unter Harnstoffbildung ablaufen könnte), abläuft.

Die so hergestellte, NCO-freie Dispersion kann sofort im Anschluß an ihre Herstellung oder nach beliebig langer Zwischenlagerung beim erfindungsgemäßen Verfahren eingesetzt werden.

Die bevorzugten, für das erfindungsgemäße Verfahren als Komponente B) eingesetzten vernetzten Polyurethandispersionen weisen dispergierte Teilchen mit einem mittleren Teilchendurchmesser von 0,5 bis 3 μm auf, sedimentieren bei mehrtägiger Lagerung, sind jedoch leicht redispergierbar und bilden, beispielsweise auf einer Glasplatte aufgetragen, wasserbeständige Filme.

Grundsätzlich kann gesagt werden, daß bei der Wahl der Polymer-Dispersion solchen Typen der Vorzug gegeben wird, die weiche und elastische Filme bilden, während solche, die spröde oder sehr harte Filme bilden nur als Mischkomponente mit den genannten bevorzugten Typen zum Einsatz gelangen. Die besonders bevorzugten Polymer-Dispersionen bilden Filme einer Härte von unter Shore A 98, vorzugsweise unter Shore A 95 und besonders bevorzugt unter Shore A 90.

Als weitere, gegebenenfalls mitzuverwendende Komponente C) ist Wasser zu nennen, welches den Systemen gegebenenfalls zugesetzt wird, um einen Wassergehalt der Mischungen aus A) und B) von 20 bis 90 Gew.-%, vorzugsweise 30 bis 80 Gew.-% und insbesondere 35 bis 65 Gew.-% einzustellen. Das Wasser hat für das erfindungsgemäße Verfahren eine besonders große Bedeutung. Es ermöglicht nicht nur eine feindisperse Verteilung der Polymeren, sondern ist auch für die Füllstoffe A und gegebenenfalls für die weiteren Zusätze D ein unerläßliches Suspensions- und/oder Emulgiermittel. Da für jede Mischung der optimale Wassergehalt von vielen Parametern, die Art und deren Mengenverhältnis abhängt, ist es von großem Nutzen, in Vorversuchen diese zu ermitteln. Bei Verwendung von zuviel Wasser ist die Koagulation erschwert und ein Ausbluten der Polymerdispersionen oft unvermeidlich. Mit zu wenig Wasser werden die Füllstoffe bei der nachfolgenden Gelierung und Koagulation nicht gleichmäßig und abriebfest eingebunden.

Weitere, gegebenenfalls mitzuverwendende Hilfs- und Zusatzmittel D) sind insbesondere stabilisierend und/oder vernetzend wirkende Substanzen. Hierzu gehören beispielsweise die bekannten Kautschukhilfsmittel, die zur Vernetzung dienen können, wie beispielsweise Zinkoxid und/oder Schwefel als auch Alterungsschutzmittel zur Vermeidung von Wärme- und Lichtempfindlichkeit durch Autoxidationen bei der Einwirkung von Sauerstoff. Geeignete Alterungsmittel sind beispielsweise phenolische Verbindungen oder aromatische Amine oder Imino-thiazole, wie o-tert.-.-Butylphenol, Metallsalze von Phenolthioethern, N-Phenyl-2-amino-naphthalin, Phenothiazin und tert.-Butyl-brenz-katechin oder 5-Benzyliden-3-hexadecyl-o-phenyl-imino-thiazolidon-(4).

Zu den gegebenenfalls mitzuverwendenden Hilfsmittel D) gehören insbesondere organische Polyisocyanate, wobei hierunter im Rahmen der Erfindung sowohl die üblichen niedermolekularen Polyisocyanate wie z.B. 2,4- und/oder 2,6-Diisocyanatotoluol, 4,4'-Diisocyanatodiphenylmethan, flüssige Derivate dieses Diisocyanats oder Polyisocyanatgemische der Diphenylmethanreihe (Phosgenierungsprodukte von Anilin/Formaldehyd-Kondensaten) einschließlich deren Sumpfrückstände zu verstehen sind, als auch NCO-Prepolymere auf Basis von einfachen Diisocyanaten der zuletzt beispielhaft genannten Art und Polyhydroxylverbindungen des Molekulargewichtsbereichs 62 bis 10000, vorzugsweise 400 bis 6000. Zur Herstellung der NCO-Prepolymeren werden vorzugsweise die aus der Polyurethanchemie bekannten Polyesterpolyole und insbesondere Polyetherpolyole des zuletzt genannten Molekulargewichtsbereichs eingesetzt, wobei vorzugsweise die entsprechenden Diole als Mischkomponenten neben höher funktionellen Polyolen Verwendung finden. Tri- und höherfunktionelle Polyole werden allein oder in Mischung mit difunktionellen Polyolen zur Steuerung der Gesamtfunktionalität NCO-Prepolymeren verwendet, so daß die NCO-Funktionalität bei 2,1 bis 3,5, vorzugsweise bei 2,2 bis 2,8 liegt. Bei den genannten Polyester- und insbesondere Polyetherpolyolen handelt es sich um die aus der Polyurethanchemie an sich bekannten Verbindungen. Besonders bevorzugt sind die entsprechenden Polyetherpolyole, bei deren Herstellung Ethylenoxid und/oder Propylenoxid im Gemisch oder in beliebiger Reihenfolge eingesetzt worden sind. Oftmals ist es auch von Vorteil, hydrophil modifizierte NCO-Prepolymere einzusetzen. Hierbei kann es sich sowohl um nichtionisch-hydrophil modifizierte Prepolymere handeln, deren Hydrophilie auf einen hohen Gehalt an innerhalb von Polyetherketten eingebauten Ethylenoxideinheiten zurückzuführen ist als auch um anionisch oder kationisch modifizierte NCO-Prepolymere, die dann erhalten werden, wenn bei der Herstellung der NCO-Prepolymeren Polyhydroxylverbindungen mitverwendet werden, die ionische Zentren oder in ionische Zentren überführbare Zentren aufweisen. Derartige Polyhydroxylverbindungen sind beispielsweise Aminopolyether, die durch Alkoxylierung von Stickstoff aufweisenden Startermolekülen erhalten werden, beispielsweise die Alkoxylierungsprodukte von N-Methyldiethanolamin, Ethanolamin, Anilin oder Ethylendiamin oder Sulfonatgruppen aufweisende Polyetherdiole beispielsweise der in US—PS 4 108 814 genannten Art oder Carboxyl- oder Carboxylatgruppen aufweisende mehrwertige Alkohole wie beispielsweise Weinsäure oder Dimethylolpropionsäure bzw. deren Alkalisalze. Im Falle der Verbindung von tert. Stickstoff aufweisenden Polyhydroxylverbindungen bei der Herstellung der NCO-Prepolymeren kann die Überführung der tert. Stickstoffatome in Ammoniumgruppe beispielsweise durch anschließende Quaternierung erfolgen, während die Überführung der gegebenenfalls zunächst vorliegenden Carboxylgruppen in Carboxylatgruppen durch Einwirkung von Basen, beispielsweise von Triethylamin im Anschluß an die Prepolymerbildung erfolgen kann.

Die gegebenenfalls mitzuverwendenden Polyisocyanate weisen im allgemeinen eine (mittlere) Funkitionalität von mindestens 2, vorzugsweise von mindestens 2,1 und insbesondere von mindestens 2,5 auf. Im Falle der bevorzugten Verwendung von NCO-Prepolymeren weisen diese einen NCO-Gehalt von 2 bis 12 Gew.-%, vorzugsweise von 2,5 bis 8 Gew.-% auf.

Unter gewissen Voraussetzungen können auch direkt alle zur NCO-Prepolymerherstellung erforderlichen Ausgangskomponenten bei der vollkontinuierlichen Produktion der erfindungsgemäßen gefüllten Trägermassen eingesetzt werden, d.h. die separate NCO-Prepolymerherstellung und Zwischenlagerung ist nicht zwingend erforderlich. Es hat sich bei dieser "in situ"-Prepolymerherstellung als ausreichend erwiesen, wenn vor allem die nieder- oder höhermolekularen Polyole, insbesondere die bevorzugt verwendeten Polyetherpolyole und gegebenenfalls weiteren H-acide Kettenverlängerungsmittel in einem Durchflußmischer mit bevorzugt aromatischen Di- und Polyisocyanaten kurzzeitig, z.B. etwa nur 10 bis 60

EP 0 209 790 B1

Sekunden bei erhöhten Temperaturen von 50 bis 120°C, bevorzugt 80 bis 100°C, weitgehend zur Reaktion gebracht werden.

Wenn der Isocyanatgehalt nach dieser kurzen Zeit unter 50%, bevorzugt unter 25%, über dem bereichneten Wert für NCO-Prepolymerumsetzung liegt, wirkt sich die unvollständige NCO-Prepolymerbildung nicht nachteilig auf die Eigenschaften der damit hergestellten Trägermassen aus, andererseits ist diese Vorgehensweise für einen vollkontinuierlichen Produktionsablauf von großem Vorteil, insbesondere wenn NCO-Prepolymere mit begrenzter Lebensdauer oder starker Erhöhung der Viskosität beim Lagern eingesetzt werden sollen. Solche NCO-Prepolymere sind z.B. solche, welche gewisse Mengen an tertiär Aminogruppen enthaltenden Verbindungen, z.B. tertiär aminhaltige Di- oder Triole, eingebaut enthalten.

Die beispielhaft genannten Polyisocyanate werden, falls überhaupt, in Mengen von bis zu 25, vorzugsweise von bis zu 15 Gew.-%, bezogen auf die Trockensubstanzen der Komponenten A) und B) eingesetzt. In diesem Zusammenhang muß jedocb hervorgehoben werden, daß im Falle der Verwendung von fossilen Lignozellulosepulvern, insbesondere von Torf und/oder Braunkohlepulvern als Komponente A) in Kombination mit ionisch modifizierten Polymerdispersionen als Komponente B) auf die Verwendung von nichtionischern oder kationischen NCO-Prepolymeren vorzugsweise ganz verzichtet wird oder nur neidermolekulare di- und/oder trifunktionelle Polyisocyanate in untergeordneten Mengen eingesetzt werden. Die organischen Polyisocyanate werden, falls sie in Wasser nicht dispergierbar sind in der Polymer-Dispersion emulgiert oder, falls sie hydrophile Zentren aufweisen und daher in Wasser dispergierbar sind vorzugsweise als wäßrige Emulsion eingesetzt.

Falls ionisch modifizierte Polymerdispersionen B) und ionisch modifizierte Polyisocyanate, insbesondere NCO-Prepolymere eingesetzt werden, ist es nicht unbedingt erforderlich, daß diese Ausgangsmaterialien in gleichem Sinne geladen sind. So ist es beispielsweise möglich, anionische Polymerdispersionen mit kationischen NCO-Prepolymeren zu kombinieren oder umgekehrt, so daß eine Ampholytbildung stattfindet, bzw, wobei das kationische NCO-Prepolymer als Koagulanz für die Dispersion dient.

Zur Durchführung des erfidnungsgemäßen Verfahrens werden die Komponenten A) und B) und gegebenenfalls C) und D) bei 10 bis 70°C, bevorzugt 20 bis 40°C, miteinander vermischt, wobei im Falle der Verwendung von saure Gruppen aufweisenden Füllstoffen, beispielsweise von Huminsäuren aufweisender Braunkohle oder von Huminsäure aufweisendem Torf vorzugsweise tert. Stickstoff aufweisende Polyisocyanate mitverwendet werden, wodurch durch Humatbildung die Wasserlöslichtkeit der Huminsäuren herabgesetzt oder unter bunden wird. Bei dieser Arbeitsweise werden vorzugsweise anionisch modifizierte Polymerdispersionen als Komponente B) verwendet. Die bei der Durch mischung der Ausgangskomponenten anfallenden Gemische weisen bei Mitverwendung von Schaumstoffen vorzugsweise einen Gehalt an Trockensubstanz von 35 bis 120 kg/m$^3$, ohne Mitverwendung von Schaumstoffen von 150—350 kg/m$^3$ Suspension auf. Im Falle der Mitverwendung von spezifisch schweren Füllstoffen, wie beispeilsweise Magnetit, liegen diese Werte mit vorgefertigten Schaumstoffanteilen vergleichbar niedrig, ohne Schaumstoffe jedoch bis etwa 400 kg/m$^3$ Suspension. In vielen Anwendungsfällen, beispielsweise bei der biologischen Abwasserreinigung, müssen die Trägermassen jedoch wenigstens zeitweise in Bewegung gebracht und gehalten werden. Daher müssen derartige, spezifisch schwere Füllstoffe mit Gehalten an Trockensubstanz über 150 kg/m$^3$ Suspension relativ feinteilig, vorzugsweise mit einem Teilchendurchmesser von unter 5 mm oder vorzugsweise unter 2 mm eingesetzt werden.

Im Anschluß an die Durchmischung erfolgt die Koagulation der Polymerdispersion B). Hierbei handelt es sich um einen komplizierten kolloidchemischen Vorgang, der von vielen Parametern beeinflußt wird und mehr oder weniger schnell in mehreren Stufen erfolgt. Die Koagulation kann sowohl bei Raumtemperatur durch Zugabe eines geeigneten Koagulierhilfsmittels als auch, im Falle der Verwendung von hitzelabilen Dispersionen B) durch eine Wärmebehandlung bei 40 bis 100°C, vorzugsweise 60 bis 100°C erfolgen. Geeignete Koagulierungshilfsmittel (Flöckungsmittel bzw. Koagulantien) sind im Falle der Verwendung von ionisch modifizierten Dispersionen Elektrolyte, die den Mischungen vorzugsweise in Form von verdünnten, d.h. 0,5 bis 5-gew.-%igen, bevorzugt 1—2%igen wäßrigen Lösungen einverleibt werden. Geeignete Elektrolyte sind beispielsweise Natriumchlorid, Magnesiumchlorid, Magnesiumsulfat, Calciumchlorid oder Trinatriumphosphat. Auch stark verdünnte Mineralsäuren wie Schwefel-, Salpeter- oder Salzsäure oder auch Carbonsäuren wie Essig- oder Ameisensäure sind als Flockungsmittel geeignet. Ebenfalls geeignet ist der Zusatz von gegebenenfalls höher konzentrierten, etwa 10—30 gew.-%igen, entgegengesetzt geladenen Polymer-Dispersionen im Anschluß an die Herstellung der Gemische, wobei erfindungsgemäße Trägermassen mit Ampholytcharakter resultieren. Oftmals ist es auch zweckmäßig, die Koagulation sowohl durch Zusatz eines Flockungsmittels als auch durch eine Wärmebehandlung herbeizuführen.

Überraschenderweise haben auch nichtionische, nieder- oder höhermolekulare Polyisocyanate, insbesondere auf anionische Polymerdispersionen einen vorteilhaften Einfluß bezüglich der Koagulation, so daß vielfach schon bei Raumtemperatur oder wenig erhöhter Temperatur eine gut steuerbare allmähliche Gelierung und Koagulation auch ohne konventionelle Koagulierungsmittel erfolgt.

Im Falle der Anwendung einer Temperaturbehandlung zur Koagulation wärmesensibler Dispersionen kann diese beispielsweise in einem Heizschrank, gegebenenfalls unter reduziertem Druck bei Temperaturen von 80 bis 100°C oder im Wärmeluftkanal bei 60 bis 100°C, vorzugsweise 85 bis 97°C

9

erfolgen, wobei das Wasser gegebenenfalls teilweise oder in manchen Fällen nahezu vollständig entfernt wird. Eine besondere Art der Wärmekoagulatin ist die Einwirkung von Infrarotstrahlen oder von Mikrowellen. Im Falle der Einwirkung von Mikrowellen werden oftmals die in den Füllstoffen fein verteilten Polymerdispersionen innerhalb von 20—60 Sekunden in einen in Wasser unlöslichen Film überführt, der die Füllstoffe abriebfest bindet. Polymerträgermassen mit einem relativ niedrigen spezifischen Gewicht, wie sie beispielsweise bei Mitverwendung vorgefertigter PUR-Schaumstoff-Granulate erhalten werden, können zur Vervollständigung der Koagulation und gegebenenfalls gleichzeitigen Windsichtung zur Abtrennung bestimmter Korngrößen mit Hilfe eines heißen Luftstromes in einen Zyklon geleitet werden, wobei in kurzer Zeit Produkte geringer Restfeuchte erhalten werden.

Während des Koagulationsvorganges darf das Gemisch nur noch mäßig bewegt werden, falls keine porösen Schaumstoffteilchen als Füllstoff A) verwendet werden. Vorzugsweise sollte die Bewegung des Gemischs während der Koagulation ganz unterbleiben, damit keine Füllstoffprimärteilchen zurückbleiben, die nicht von der koagulierenden Dispersion umhüllt sind. Lediglich bei Mitverwendung von offenzelligen Schaumstoffen ist dieser Punkt weniger kritisch, so daß Koagulation überraschenderweise sogar unter lebhafter Bewegung der Mischung erfolgen kann, so daß die Schaumstoffteilchen untereinander nicht verkleben.

Eine schockartige Koagulation der Dispersion sollte jedoch besonders bevorzugt nur in ruhendem Zustand der Gesamtmischung erfolgen, wobei darauf zu achten ist, daß das wäßrige Bindemittel vorher gleichmäßig verteilt wurde.

Zur Durchführung der Koagulation wird somit dem gemisch aus den Komponenten A) und B) und gegebenenfalls C) und D) im Falle einer ionischen Koagulation das Flockungsmittel durch Durchmischen einverleibt, worauf anschließend von der genannten Ausnahme abgesehen, das Reaktionsgemisch ohne weitere Bewegung sich selbst überlassen wird. Die Koagulation selzt je nach Art der Polymerdispersion und ja nach Art des Füllstoffes und des Flockungsmittels innerhalb eines Zeitraums von 30 Sekunden bis 10 Minuten, bevorzugt unter 3 Minuten, nach Zugabe des Flockungsmittels ein und kann gewünschtenfalls, wie ausgeführt, durch eine gleichzeitig erfolgende Wärmebehandlung beschleunigt werden. Im Falle der vorstehend beschriebenen Verwendung von ionischen NCO-Prepolymeren, die eine andersartige Ladung aufweisen als die Polymerdispersion B) erfolgt die Zugabe derartiger ionischer Prepolymerer vorzugsweise nach Herstellung der Gemische aus A) und B) und gegebenenfalls C) und D) (mit Ausnahme der genannten Prepolymeren), so daß die durch die Prepolymeren mit Wasser bewirkte Vernetzung (unter Ausbildung von Polyharnstoffen) mit der durch ihre ionische Ladung besonders schnell hervorgerufenen Koagulation der Polymerdispersion einhergeht. Falls die Polymeren H-azide Gruppen aufweisen, kann der Polyurethanharnstoff sogar über homöopolare Bindungen mit dem Polymer vereinigt werden.

Die Herstellung der Mischungen aus den Komponenten A), B) und gegebenenfalls C) und D) und auch das Einmischen des Flockungsmittels erfolgt in beliebigen Mischaggregaten, beispielsweise in einem Schneckentrog oder unter Verwendung eines Kneters oder mit pflugscharänlichen Werkzeugen ausgestatteten Mischern. Nach der Koagulation fallen die erfindungsgemäßen Trägermassen in stückiger Form oder als Granulat an und können, gewünschtenfalls, mechanisch weiter zerkleinert werden. Oftmals ist es angezeigt, gebenenfalls mitverwendete ionische Koagulationshilfsmittel durch Auswaschen der anfallenden Trägermassen mit Wasser zu entfernen, falls diese die Lagerstabilität beeinflussen oder bei der späteren Verwendung stören könnten.

Die Durchführung des erfindungsgemäßen Verfahrens kann sowohl kontinuierlich als auch diskontinuierlich erfolgen.

Eine ganz spezielle Methode der Herstellung der erfindungsgemäßen Trägermassen besteht im Falle der Verwendung von Weichschaumstoffen als Komponente A) darin, daß man sofort nach Durchmischen der Ausgangskomponenten und noch vor Einsetzen der Koagulation das Mischgut, gegebenenfalls zusammen mit dem Koagulationshilfsmittel in einer geeigneten Vorrichtung, beispielsweise einem kastenförmigen Metallbehälter auf einen Bruchteil seines Schüttvolumens zusammenpreßt und die Koagulation unter Druck und gegebenenfalls Wärmeeinwirkung durchführt, um so eine erfindungsgemäße Trägermasse einer vorgegebenen Dimension und Dichte zu erhalten.

Diese Methode ist von besonderem Interesse, wenn besonders leichte Polyurethanblockschaumstoffe als Komponente A) eingesetzt werden. Ausgehend von Schaumstoffabfällen der Dichte 25 kg/m$^3$ können so beispielsweise unter Mitverwendung von nur 15 Gew.-% Polymer-Dispersion (bezogen auf Feststoff) Verbundschaumstoffe mit einem auf das 6 bis 10-fach gesteigerten Raumgewicht hergestellt werrden. Die gepreßten Formschaumstoffe können in Lagen beliebiger Dicke oder in Folien geschnitten oder in beliebiger Größe in Zerhackern zerkleinert werden.

Die Herstellung von erfindungsgemäßen Trägermassen, die ohne Mitverwendung von Schaumstoffen als Komponente A) bzw. als Teil der Komponente A) hergestellt werden, führt, wie bereits angedeutet, zu Trägermaterialien eines erheblich höheren spezifischen Gewichts.

In wäßriger Suspension weisen die Polymerträgermassen bei 100 Volumenprozent Füllung (ohne überstehendes Wasser) einen Gehalt an Trockensubstanz (TS) von etwa 40 bis 350 kg/m$^3$ auf. Die mit Schaumstoffen hergestellten Typen haben in wäßriger Suspension einen bevorzugten TS-Gehalt von 40—95 kg/m$^3$, vergleichbare Typen mit Ausnahme der mit Torf hergestellten, ohne Schaumstoffanteile einen TS-Gehalt von 150—350 kg/m$^3$. Weger der Einsparung an Ausgangskomponenten (bei gleichen Volumina Suspension), der vielfach größeren Aufwuchsfläche für Mikroorganismen und der leicht handzuhabenden und beliebig variierbaren Verwendung in einem Fest-, Fließ- oder Wirbelbett haben die erfindungsgemäßen, hochgefüllten Trägermassen mit gut ausgebildeter, offenzelliger Struktur den besonderen Vorzug. Wie oben ausgeführt, schwankt die Dichte der erfindungsgemäßen Trägermassen in Abhängigkeit von der Art und Mengenrelation der Ausgangskomponenten, ihrem Wassergehalt bei der Herstellung, von dem beschriebenen, gegebenenfalls angewendeten Kompressionsgrad und insbesondere in Abhängigkeit vom spezifischen Gewicht der eingesetzten Füllstoffe A) innerhalb weiter Grenzen und liegt mit einem mittleren Wassergehalt von 50 Gew.-% im allgemeinen bei einem Schüttgewicht von 300 bis 700, vorzugsweise 400 bis 550 kg/m$^3$. Die Wasseraufnahmefähigkeit der Trägermassen (WAF, Erklärung nachstehend in Beispielteil) liegt bei 30 bis 97 Gew.-% Wasser, bezogen auf Trockengesamtgewicht, vorzugsweise über 85 Gew.-%. Die mittlere Teilchengröße der im allgemeinen in stückiger Form oder als Granulate anfallenden Verfahrensprodukte liegt in Abhängigkeit von der Teilchengröße der eingesetzten Füllstoffe A) und in Abhängigkeit von der Agglomeratbildung während der Durchmischung ohne Verwendung von Schaumstoffen bei 1—10 mm, vorzugsweise unter 5 mm. Die weitere Agglomeratbildung kann durch rasche Umhüllung der Füllkörper mit der Polymerdispersion weitgehend vermieden werden.

Die mit vorgefertigten Schaumstoffen entsprechend leichteren Polymerträgermassen können auch in wesentlich größeren Teilchen anfallen und verwendet werden. In den erfindungsgemäßen Trägermassen liegen die Füllstoffe, insbesondere die bevorzugt eingesetzten Schaumstoffe in bezüglich ihrer ursprünglichen Struktur und physikalischen Eigenschaften völlig veränderter Form vor. Die Zellstruktur der eingesetzten, bevorzugt weichelasstischen PUR-Schaumstoffe ist, wie elektronenmikroskopische Aufnahmen deutlich zeigen, durch deie erfindungsgemäße Koagulation von Polymeren aus wässriger Phase wesentlich verstärkt. Die koagulierten Polymeren umhüllen die Zellstege der Schaumstoffe und werden somit ein integrierter Bestandteil derselben unter matrixartiger, abriebfester Einbindung bevorzugt verwendeter Füllstoffe, wie Braunkohlenstaub, so daß die Zellräume zwar verkleinert oder teilweise völlig ausgefüllt werden, aber hochelastisch bleiben und mit einem auf das 2-5-fach erhöhten spezifischen Gewicht, bezogen auf den eingesetzten Schaumstoff. Aus den unbrauchbaren, aufschwimmenden Abfallschaumstoffen werden dadurch im Wasser langsam absinkende Trägermassen mit erhöhter spezifischer Festigkeit, sehr hohem Wasseraufnahmevermögen und durchdringbaren Aufwuchsflächen und Schutzräumen für Mikroorganismen.

Zur Regulierung des spezifischen Gewichts können bei der Herstellung dieser hochgefüllten Polyurethan(harnstoff)massen auch anorganische Füllstoffe in feinstverteilter Form verwendet werden, wodurch das notwendige spezifische Gewicht für die Klärflüssigkeit eingestellt und gegebenenfalls die Sauerstoffübertragung auf die Bakterien begünstigt werden kann.

Die erfindungsgemäßen, wassergequollenen, hydrophilen Träger stellen in bevorzugten Einstellungen weichelstische, sich meist trocken anfühlende, abriebfeste Teilchen dar, welche sich in Wasser suspendieren lassen dund dort schweben oder bevorzugt langsam absinken.

Es war nicht vorauszusehen, daß die mit Füllstoffen wie Braunkohle hochgefüllten gegebenenfalls Schaumstoffe enthaltenden Polymeren als Träger mit stark hydrophilen Eigenschaften in homogener oder zelliger Struktur ausreichend abriebfest hergestellt werden können auf die biologische Klärung einen so günstigen Einfluß nehmen, obwohl die Füllstoffe aktivier Art, wie z.B. Braunkohle, innerhalb der als Matrix fungierenden Polymermasse eingebettet liegen und sich die Biomasse de Bakterien zunächst in der äußeren, kohärenten wäßrigen Phase befindet, und erst dort von außen nach innen aufwächst.

Die erfindungsgemäß eingesetzten Träger eignen sich für die meisten der gebräuchlichen Verfahren zur biologischen, aeroben oder anaeroben Klärung von Abwässern sowohl in industriellen als auch in kommunalen Kläranlagen.

Nach dem stand der Technik ist die Verwendung von Weichschaumstoffen auf Polyurethanbasis als Träger in biologischen Reinigungsverfahren von Abwässern beschrieben. Es hat sich jedoch gezeigt, daß Leichtschaumstoffe üblicher Raumgewichte von 15 bis 35 kg/m$^3$, wie sie als Schaumstoffabfall im Handel sind, keinesfalls alleine als Trägermaterialien in Klärschlammbecken mit Erfolg benutzt werden können. Derartige Schaumstoffe schwimmen immer auf, führen zu Verstopfungen und sonstigen widrigen Charakteristiken ihres Verhaltens in Kläranlagen. Polyurethan-Weichschaumstoffe mit relativ hohen Gerwichten von ca. 90 kg/m$^3$ sind zwar etwas günstiger, jedoch auch noch nach Monaten (siehe Vergleichsbeispiele) zu einem erheblichen Teil an der Oberfläche der Klärbecken schwimmen. Auch führen sie je nach Schichtdicke der Schaumstoffe zu Verstopfungen des Ablaufs oder werden sogar mit dem Ablauf ausgetragen. Derartig aufschwimmende Schaumstoffe sind für die Biomasse weitgehend unwirksam und ergeben vielfach wohl unüberwindliche technische Schwierigkeiten. Auch die Zugabe von Aktivkohle zu den Schaumstoffen bringen keine Vorteile, selbst dann nicht, wenn dieser vorher in den Schaumstoff mechanisch eingequetscht werden.

Die biologische Umwandlung organischer Verunreinigungen mittels Bakterien unter Sauerstoffversorgung in überwiegend aus Kohlehydraten und Proteinen bestehenden, durch Vermehrung sich bildenden Baktierenmassen, in $CO_2$ und Wasser, ferner gegebenenfalls Nitrate, wird als aerobe Abwasserreinigung bezeichnet.

Die Umwandlung organischer Verunreinigungen, bevorzugt Kohlehydrate, Eiweiß und Fette ohne Sauerstoffzufuhr mittels säurebildender Bakterien, sulfatreduzierender Bakterien und methanerzeugender Bakterien unter Bildung von Schwefelwasserstoff, Kohlendioxid und insbesondere Methan wird als anaerobe Abwasserreinigung bezeichnet.

11

# EP 0 209 790 B1

Die erfindungsgemäßen, hochgefüllten, hoch wasseraufnahmefähigen Polymerträgermassen bewirken sowohl im ruhenden als auch im bevorzugt bewegten Zustand die verbesserte biologische Reinigung von Abwässern, besonders überraschend auch von Abwässern mit sehr geringer Konzentration an Schadstoffen, beispielsweise unter 500 mg/l, was für die Endreinigungsstufe in Kläranlagen zum einwandfreien Ablauf von geklärtem Wasser sehr wichtig ist.

Die erfindingsgemäße Reinigung kann deshalb in der ersten und/oder in nachgeschalteten weiteren Belebungsstufen erfolgen, indem man die Träger an beliebiger Stelle einem oder mehreren Kombinierten Belebtschlammbecken zuführt. Da die erfindungsgemäßen, Polymerträgermassen als Träger trotz ihres relativ geringen Anteils an Polymeren in hohem Grade in Wasser abriebfest sind, können sie sowohl in Klärbecken mit hoher Turbulenz als auch in Behältern mit nicht oder nur wenig bewegten Klärschlämmen eingesetzt werden, d.h. die hochgefüllten Polymerträgermassen können in entsprechenden Wirbel-, Fließ- oder Festbettanordnungen verwendet werden.

Durch Einleiten von Luft und/oder (reinem) Sauerstoff entsteht bei den in großem Umfang betriebenen aeroben Klärverfahren eine beträchtliche Turbulenz. Dadurch werden in einem sogenannten flüssigen Wirbelbett die hochgefüllten Polymerträgermassen und der Belebtschlamm lebhaft in Bewegung gehalten. Trotzdem bildet sich auf der Oberfläche und teilweise auch im Innern des hochgefüllten Polymers ein Bakterienrasen, der die im experimentellen Teil beschriebene, überraschend erhöhte Reinigungs- leistung erzielt. Der im Polymer inkorporierte Füllstoff hat in mehrfacher Hinsicht einen vorteilhaften Einfluß auf das verbesserte Entsorgungsverfahren. Je nach Art der Füllstoffe und Art der Polymer-Matrix werden die mechanische Festigkeit und die Hydrophilie des Trägermaterials verbessert und insbesondere überraschend die biologische Assimilationsfähigkeit der im Abwasser gelösten organischen Stoffe wesentlich erhöht. Außerdem ist der im Polymerträger eingebundene Füllstoff oder die Füllstoffmischung gleichzeitig ein Regulator für die Einhaltung optimaler, spezifischer Gewichte der für Wasser durchlässigen, erfindungsgemäß eingesetzten Träger, so daß in den üblichen, etwa 4 bis 12 m hohen, stark gefüllten Belebtschlammbecken eine gleichmäßige Verteiling der Träger mit schwacher Sinktendenz oder die Erhaltung eines schwebenden Zustandes möglich ist. Dies ist für die meisten der heute von den Kommunen oder der Industrie betriebenen, biologisch arbeitenden Kläranlagen und besonderer Bedeutung oder sogar eine verfahrenstechnische Voraussetzung.

Wie bereits erwäht, können im Gegensatz dazü übliche, nicht erfindungsgemäß modifizierte schaumstoffartige Kunststoffe mit makroporöser Struktur, einschließlich Polyurethan-Schaumstoffe, in der praktizierten Kläranlagentechnik nicht so wirkungsvoll und nicht wie erforderlich in mehrjährigem Dauerbetrieb gehandhabt werden, da diese Schaumstoffe selbst im Fall relativ hoher Raumgwichte von ca. 90 kg/m$^3$ noch nach Monaten zu erheblichen Anteilen an der Oberfläche der Klärbecken schwimmen und technische Schwierigkeiten (z.B. Verstopfungen) hervorrufen. Ganz unpraktikabel hat sich die Verwendung von Schaumstoffen üblich niedriger Raumgewichte von 20—35 kg/m$^3$ (sogenannte Abfallschaumstoffmischungen) heausgestellt. Diese Schaumstoffe schwimmen auch unter kräftigem Rühren an der Wasseroberfläche.

Wie bereits ausgeführt, werden in einer speziellen Ausführungsform der Erfindung die Polymerträgermassen mit ihren Füllstoffen und gegebenenfalls Zusätzen so eingestellt, daß sie im Belebungsbecken der Kläranlage sofort oder innerhalb weniger Stunden absinken und trotz ausreichender Luft- und Sauerstoffdurchflutung als Trägermaterialien mit der nach einer gewissen Zeit beträchtlichen Menge anhaftenden Biomasse ein von sauerstoffhaltigem Gas durchströmtes Wirbel-, Fließ- oder Festbett mit überstehender Träger-freier Wasserschicht bilden, das im Bedarfsfall, z.B. zum gelegentlichen oder kontinuierlichen Abführen von Überschußschlamm durch eine entsprechend starke Begasung verändert werden kann. Die erfindungsgemäß eingesetzten, hochgefüllten, hydrophilen Polymerträgermassen werden auch hierbei nicht ausgetragen.

Neben der weitverbreiten aeroben biologischen Reinigung von Abwässern hat, insbesondere bei stark kohlehydrathaltgen Abwässern, beispielsweise in der Lebensmittel oder Zellstoffindustrie, auch die anaerobe Abwasserreinigung eine große technische Bedeutung. Die erfindungsgemäß hergestellten und so verwendeten Polymerträgermassen sind hervorragend geeignet, biologisch auch sehr hohe Schadstoffkonzentrationen von über 25.000 mg/l in einer Stufe der Abwasserreinigung zu bewältigen, oder auch bisher kaum abbaubare, organische Chlorverbindungen zu eliminieren. In manchen Fälen ist eine kombinierte anerobe und aerobe biologische Abwasserreinigung besonders wirkungsvoll. Auch hier sind die erfindungsmäßen, hochgefüllten, hydrophilen Träger günstig einzusetzen.

Der Grad der Hydrophilie wird bei den erfindungsgemäßen, hochgefüllten Polymerträgermassen bevorzugt so eingestellt, daß eine hohe Wasseraufnahme innerhalb von Stunden oder wenigen Tagen unter starker Quellung erfolgt oder eine größere Wassermenge bereits bei der Herstellung der Polyurethanmassen als disperse Phase vorliegt und somit die Träger bereits voll gequollen sind. Bei der anaeroben Klärtechnik wie auch der aeroben Abwasserreinigung können die erfindungsgemäßen Produkte größere Mengen gasförmiger Produkte wie Kohlensäure, Methan oder Schwefelwasserstoff gut entweichen lassen.

Der "in situ"-Einbau von Mikroorganismen in Polyurethane oder andere Kunststoffe ist, wie schon erwähnt, bei den für die Abwasserreinigung verwendeten Biomassen auch unter sehr schonenden und technisch aufwendigen Bedingungen ohne Verlust an vermehrungsfähigen Bakterien und starker Minderung der Bioaktivität praktisch nicht möglich und deshalb nicht bevorzugt. Bei der

12

erfindungsgemäßen Verwendung erübrigt es sich auch, weil in den ausreagierten, hochgefüllten Polymerträgern überraschenderweise sogar im Wirbelbett ein großer Teil der Bakterienkulturen einen festen Halt finden und sogar in die hochgefüllten, stark quellfähigen, suspendierten Trägermassen eindringen können und gegen mechanische Schädigung somit geschützt sind, Die Bakterien sitzen am gleichen Ort, an dem durch Adsorption die hochgefüllten Polymerträger eine erhöhte Konzentration an gelösten Schadstoffen bewirken.

Die Abbauleistung und Reinigungswirkung, d.h. die Verbesserung der Abwassergüte, nach dem erfindungsgemäßen Verfahren beschränkt sich nicht nur auf eine deutliche Verringerung des chemischen Sauerstoffbedarfs (CSB-Werte), sondern begünstigt auch eine drastische Reduzierung der Daphnien-Toxizität und Fisch-Toxizität durch Eliminierung sonst schwer oder nicht abbaubarer toxischer Verbindungen, was zweifellos von mindestens ebenso großer Bedeutung ist. Außerdem wird eine weitgehende Beseitigung des in vielen Kläranlagen auftretenden üblen Geruchs und eine zusätzliche deutliche Farbaufhellung des geklärten Abwassers erreicht. Darüber hinaus kann die Kapazität einer vorhandenen biologischen Kläranlage deutlich gesteigert werden.

Die erfindungsgemäßen, hochgefüllten Polymerträgermassen als Träger verbessern auf zweierlei Weise die Reinigungsleistung biologischer Kläranlagen ganz entschieden. Durch die erfindungsgemäßen Träger können nicht nur allgemein Abwasserinhaltsstoffe an der Oberfläche der Träger aufkonzentriert werden, es können vielmehr gezielt auch Stoffe, beispielsweise Chlorkohlenwasserstoffe wie Ethylenchlorid, aus Abwässern angereichert werden, die sonst in ihrer gelösten Form und aufgrund ihrer geringen Konzentration neben anderen, gut abbaubaren Verbindungen von den Mikroorganismen des Belebtschlamms nicht verwertet werden können. Die Substratkonzentration solcher Verbindungen wird dafür auf den für den biologischen Abbau notwendigen Wert angehoben. Gleichzeitig siedeln sich auf den hochgefüllten Trägern Mikroorganismen an und vermehren sich aufgrund des gut angereicherten Substratangebotes optimal. Adsorptionsflächen für Substrate, d.h. für die im Abwasser enthaltenen organischen Verbindungen geringerer Konzentrationen werden nach der Umwandlung durch Bakterien wieder freigesetzt. Die Vorgänge Adsorption und Verwertung von gelösten Abwasserinhaltsstoffen an den Trägern, auf welchen die Mikroorganismen aufgewachsen sind, laufen kontinuierlich ab. Es bildet sich ein Gleichgewichtszustand zwischen Adsorption und Anreicherung der im Wasser gelösten Stoffe und dem biologischen Abbau durch die an der Oberfläche der Träger gleichfalls angesiedelten Mikroorganismen. Dadurch wird eine fortlaufenden Regeneration der Oberfläche erreicht. Ebenso bildet sich in Abhängigkeit vom Substratangebot ein Gleichgewicht zwischen Biomassen-Wachstum auf den hochgefüllten Polymerträgermassen und Elimination der Stoffe, so daß ständig eine erhöhte Biomassen-Aktivität an den Trägern erhalten bleibt.

Es ist möglich, mit Hilfe der erfindungsgemäßen Träger die Belebtschlammkonzentration in biologischen Kläranlagen deutlich zu erhöhen, zumeist mindestens zu verdoppeln und damit die Raum-Zeit-Belastung zu vervielfachen, so daß die Kapazität vorhandener Kläranlagen erheblich erhöht wird oder bei Neuanlagen kleinere Beckenvolumina ausreichen.

Eine einfache technische Anwendung der Träger besteht in der Zugabe in ein konventionelles, biologisches Belebungsbecken. Durch die Gas/Flüssigkeitsströmung werden die Trägerteilchen in Schwebe gehalten und im Belebungsraum gleichmäßig verteilt. Die hochgefüllten Polyurethan-Träger können aufgrund ihrer extrem hohen Abriebfestigkeit problemlos auch mit Oberflächen-Belüftern ausgestatteten Belebungsbecken eigesetzt werden.

Besonders vorteilhaft wirkt sich der Einsatz bei der Nitrifizierung und Denitrifizierung von Abwässern aus, da die hierfür notwendigen Mikroorganismen langsam und vorzugsweise auf Aufwuchsflächen wachsen.

Für den aeroben Bereich der Abwässerreinigung können diese Anlagen als Wirbelbett-Reaktoren oder als Festbett-Reaktoren betrieben werden. Die Durchströmung im Festbett kann sowohl von unten nach oben als auch von oben nach unten erfolgen. Ebenso ist der Betrieb als Tropfkörper möglich. Die hochgefüllten, bevorzugt zelligen Polymerträgermassen werden wegen ihrer besonders großen Oberfläche auch als Bewuchsfläche (Tauchtropfkörper) vorteilhaft eingesetzt.

Für die Anwendung im Bereich der Abwasserreinigung stehen für die hochgefüllten, erfindungsgemäß einsetzbaren Trägermassen mehrere Möglichkeiten zur Auswahl. Die Anlagen können, wie bereits erwähnt, sowohl im Fließ- oder Wirbelbett als auch im Festbett betrieben werden. Bei einer Festbett-Fahrweise können die hochgefüllten anionischen Polymerträgermassen als Granulat oder als feste Einbauten eingesetzt werden, wie z.B. in Form von ausgerollten Matten oder vorgefertigten Einschüben. Auch hier kann die Durchströmung im Festbett sowohl von unten nach oben wie auch umgekehrt erfolgen. Die Betriebsweise wird im allgemeinen von der jeweiligen Beschaffenheit und Besonderheit des Abwassers abhängig durchgeführt.

Der technische Fortschritt der erfindungsgemäßen Verwendung ist fernerhin und insbesondere darin zu sehen, daß mit den hochgefüllten Polymerträgermassen als Träger sogar konventionell biologisch vorgereinigte Abwässer wirkungsvoll noch von solchen Schadstoffen befreit werden können, die einen relativ sehr großen Anteil schwer abbaubarer, organischer Restmengen enthalten, den die Mikroorganismen in einer konventionellen biologischen Anlage aufgrund des Verdünnungsgrades ihrer langsamen Vermehrung und Gefahr des Auswaschens nicht mehr abbauen können.

Mit den erfindungsgemäßen Trägern kann auch eine Reinigung von Abluft von organischen Bestandteilen, z.B. Abluft von Kläranlagen oder der Abluft von Produktionsprozessen organischer Verbindungen, durch ein- oder mehrfaches Durchsaugen oder in Kontakt mit feuchten oder nassen, bzw. in Wasser suspendierten, hochgefüllten Polymerträgern erfolgen.

Beispielsweise wird durch eine oder mehrere, in Reihe geschalteten Säulen, die mit dem erfindungsgemäß einsetzbaren Trägermassen in eine auf 50 bis 80 Vol.-% verdichteten Packung gefüllt sind und gegebenenfalls mit geeigneten Mikroorganismen-Suspensionen für den biologischen Abbau versetzt werden, gleichzeitig (z.B. von oben) Abluft eingeleitet und mit Wasser berieselt. Nach Verweilzeiten von etwa 5 bis 60 Sekunden erfolgt bereits eine hohe biologische Eliminierung von organischen Schadstoffen, die nach einer relativ kurzen Startperiode zu einer lebhaften Vermehrung von abbauenden Mikroorganismen führt. Auch bei diesem ökonomisch günstigen Prozeß wird wie in wäßrigen Suspensionen, in einem physikalisch-biologischem Gleichgewicht die Adsorption der Schadstoffe und deren Abbau in Gegenwart eines Feuchtigkeitsfilms auf und in der Trägermasse gleichzeitig und am gleichen Ort vollzogen. Der Überschuß an zuwachsenden Mikroorganismen kann durch gelegentliches, ein- oder mehrmaliges Füllen der Bioreaktor-Säulen mit Wasser und kräftiges Durchblasen von Luft abgezogen werden.

Die Entsorgung der erfindungsgemäß vewendeten Trägermassen ist wegen ihres inerten Charakters problemlos. So können diese beispielsweise in Kläranlagen, in denen der Überschußbelebtschlamm in einem Wirbelschichtofen verbrannt wird, während eines jahrelangen Langzeitgebrauchs gegebenenfalls mit dem Überschußbelebtschlamm ausgetragen werden und als Energieträger mitverbrannt werden. Im allgemeinen ist ein Austausch des gesamten Trägermaterials jedoch nicht erforderlich.

Eine weitere, wichtige Verwertung liegt in ihrer Verwendung als Träger für Bakterien oder Enzyme in Biokonversionsprozessen zur Herstellung komplizierter organischer Verbindungen. Die stückigen Polymerträgermassen können von den Reaktionsbrüden oder Fermentationsbrüden durch Filtration leicht abgezogen werden, beispielsweise zur Herstellung von Zitronensäure aus Stärke zur Hydrolyse von Penicillin G mittels Acylasen zu 6-Aminopenicillansäure, ferner zur Herstellung von stereospezifischen, biologisch aktiven Verbindungen, oder zur Vergärung von zuckerhaltigen Wässern in der Rübenzuckerindustrie.

Die Verwendung der erfindungsgemäßen, bevorzugt Schaumstoffe- und -Braunkohle- oder Torfhaltigen Polymerträgermassen liegt in ihrer hochhydrophilen Eigenschaft und ist gegebenenfalls in ihrer leicht porigen Struktur begründet. Man kann sie deshalb auch als Bodenverbesserer oder Spezialwachstumsträger hydrophiler, leicht bewurzelungsfähiger Art für Pflanzen benutzen, da sie beliebige Pflanzennährstoffe enthalten können, einen sehr lange verwertbaren Wassergehalt und gegebenenfalls Düngergehalt aufweisen und leicht wieder rückquellen.

Den Trägermassen können bei der Herstellung auch Samen zugemischt werden, die dann zum Keimen gebracht werden und in z.B. Plattenformen, beispielsweise als Petersilienrasen, verwertbar sind oder in kleinstückiger Form als Setzlinge mit Polymerträgerstücken eingesetzt werden können.

Die stückigen Träger können ferner als Filtriermedium für feinteilige, emulgierte ode suspendierte Verunreinigungen, in Wasser benutzt und, beispielsweise durch Rückspülen, regeneriert werden. Eine besonders wirkungsvolle Verwendung finden die erfindungsgemäßen Träger als Adsorbentien für (Roh)Öl oder andere, nicht wasserlösliche organische Flüssigkeiten.

## Ausführungsbeispiele

1. Vorbemerkungen:

Charakterisierung der füllstoffhaltigen Polymerträgermassen:

Das erhaltene, gegebenenfalls granulierte Trägermaterial wird mit überschüssigem Wasser versetzt, 24 Stunden (bei Raumtemperatur) vollständig gequollen und nach dem Aufrühren das darüber stehende Wasser abdekàntiert. Der hieraus abgeleitete Wert, der die Gewichtsprozentmenge Wassers im und zwischen dem gequollenen Träger (füllstoffhaltigem Polymerträger) angibt, wird hier als Wasseraufnahmefähigkeit (WAF) bezeichnet (siehe nähere Erläuterung weiter unten).

Der Feststoffgehalt de so hergestellten wäßrigen Suspension des Granulats in Form eines nunmehr stark gequollenen Trägermaterials betragt (für das Beispiel 1) 59.5 g Feststoff pro Liter "Suspension" (ohne überstehendes Wasser).

Der Feststoffgehalt in einem Liter einer derartigen Suspenson (ohne überstehendes Wasser) wird als Trockensubstanz der Suspension (abgekurzt TS(S)) bezeichnet.

Das Gewicht von einem Liter dieser Suspension des stark gequollenen Trägermaterials (ohne übestehendes Wasser) wird als Suspensionsgewicht (abgekürzt SG) bezeichnet.

Aus dem Gewicht von einem Liter der Suspension (SG) und dem Wert der darin enthaltenen Trockenmasse des Trägers (TS—S) wird der Wert des sogenannten Suspensionsfaktors (F4) abgeleitet. Der Wert des Suspensionsfakters F4 minus 1 (F4-1) gibt an, die wievielfache Menge Wasse (bezogen auf Trägertrockensubstanz) in der Suspension ingesamt (als Quellwasser und als Wasser in den Zwischenräumen in oder zwischen den Trägerpartikeln) sich befindet.

Der Wert des Suspensionsfakters F 4 wird in Praxis dadurch bestimmt, daß man die Trägertrockenmasse von einem Liter einer Suspension der Träger in Wasser (ohne überstehendes Wasser) bestimmt und das Gewicht der Suspension (SG) durch das Gewicht der darin enthaltenen Trägertrockenmasse (TS(S)) dividiert:

$$F4 = \frac{SG}{TS(S)}$$

Aus diesem Wert des Suspensionsfaktors F4 kann die Wasseraufnahmefähigkeit (WAF) als Charakteristikum für die erfindungsgemäß zu verwendenden Trägermassen nach folgender Formel bestimmt werden:

$$WAF = \frac{F4\ minus\ 1}{F4} \cdot 100;\ (in\ \%)$$

Dieser Wert der Wasseraufnahmefähigkeit (WAF) in Gew.-% ausgedrückt, gibt ein anschauliches Bild für den Zusand der hochgequollenen und gegebenenfalls wasseraufnehmende Zwischenräume tragenden Trägermassen wieder, wie sie in dem gequollenen Zustand in der Kläranlage verwendet werden. Im Beispiel 1 beträgt beispielsweise der Trockensubstanzgehalt von 1 Liter der Suspension ohne überstehendes Wasser 59,5 g Feststoff. Bei einem Suspensionsgewicht von 1001 g pro Liter Suspension erreicht sich hieraus der Suspensionsfakter

$$F4 = \frac{1010}{59,5} = 16,8.$$

Ein Gewichtsteil Trockensubstanz der Trägermasse wird somit mit der 15,8-fachen Wassermenge in die beschriebene gequollene Suspensionsform überführt. Anders ausgedrückt, beträgt der Wert der Wasseraufnahmefähigkeit 15,8 durch 16,8 mal 100 = 94%.

S1. Schüttgewicht, abgetropft:
Die Trägermasse wird 24 Stunden in einer großen Überschußmenge an Wasser suspendiert, danach wird mit dieser gequollenen Masse ein Sieb mit 2 mm Sieblöchern 10 cm hoch gefüllt und 1 Stunde abtropfen gelassen; die verbleibende Füllmenge wird danach in einem Meßgefäß gewogen und auf das Schüttgewicht pro Liter umgerechnet.

S2. Schüttgewicht, ausgequetscht:
Die nach S1 abgetropfte Trägermasse wird auf einem 1 mm Sieb 5 Minuten lang einem Druck von 3 bar ausgesetzt und danach in einem Meßgefäß gewogen. Nach Umrechnung auf einen Liter wird das Schüttgewicht S2 ermittelt.

S3. Schüttgewicht, getrocknet:
Die feuchte, ausgequetschte Trägermasse wird (etwa) 1 Tag bei 100° unter Vakuum bis zur Gewichtskonstanz getrocknet und in einem Meßgefäß wie oben ausgewogen. Im oben angeführten Beispiel betragen die so bestimmten Werte von S1 bis S3:

S1 (abgetropft) 492 g/l

S2 (ausgequetscht) 214 g/l

S3 (getrocknet) 73,5 g/l.

Zur besseren Vergleichbarkeit der Werte werden noch folgende Faktoren definiert:
F1: Der Volumenfaktor, ist der Quotient aus dem Gewicht der in Wasser gequollenen, abgetropften Probe po Liter und der ermittelten Gewichtsmenge Trockensubstanz aus einem Liter wäßriger Suspension (TS(S)).

$$F1 = \frac{F1}{TS(S)}$$

F2: Der Quetschfaktor ist entsprechend der Quotient der ausgequetschten Probe pro Liter (Schüttgewicht S2) und der Trockensubstanzmenge pro Liter Suspension.

$$F2 = \frac{S2}{TS(S)}.$$

15

F3: De Quellfaktor ist der Quotient aus der Gewichtsmenge der abgetropften Probe (S1) und die nach vollständiger Entfernung des Wassers aus der abgetropften Probe ermittelten Gewichtsmenge der Trockenmasse (TS(S1)).

$$F3 = \frac{S1}{TS(S1)}$$

In den Beispielen wurden die Bestimmungen des chemischen Sauerstoffbedarfs nach DIN 38409-Teil 41 (Dezember 1980), der Fischtoxizität nach DIN 38412-Teil 15 (Juni 1982), der Daphnientoxizität nach DIN 38412-Teil 11 (Oktober 1982) und des Geruchsschwellenwertes nach den deutschen Einheitsverfahren zur Wasseruntersuchung, Loseblatt-Sammlung, Ausgabe 1982, Verlag Chemie-Weinheim, vorgenommen.

2. Zusammensetzung und Herstellugn spezieller Polyurethan-Ausgangskomponenten.

2.1. Herstellung der NCO-Prepolymeren, diskontinuierliches Verfahren für die Patentbeispiele.

Die Herstellung der NCO-Prepolymeren erfolgt in an sich bekannter Weise in einer Rührapparatur durch Erhitzen der Ausgangskomponenten (höhermolekulare Polyhydroxylverbindung, gegebenenfalls niedermolekulare Polyole, gegebenenfalls tertiären Stickstoff enthaltende Polyole und Polyisocyanate) bei Temperaturen von etwa 70 bis 90°C, bis annähernd der berechnete NCO-Gehalt erreicht ist. Zusammensetzung siehe Tabelle 1).

TABELLE 1

Zusammensetzung und charakterisierung der NCO-Prepolymeren (PP)

| Typ | Viskosität mPa.2/25°C | % NCO | Isocyanat Menge | / Art | Polyetherpolyol Menge | / Art | NM | SS | DMS |
|---|---|---|---|---|---|---|---|---|---|
| Opp | 2,400 | 5,9 | 15,3 | TDI | 46,2 | PHOBV | — | | |
| | | | | | 38,5 | PHOBL | | | |
| BOPP | 15,600 | 5,7 | 20,6 | TDI | 76,3 | PHOBV | 3,1 | 0,2 | |
| KOPP | | 2.2 | 15,3 | TDI | 46,2 | PHOBV | 3,1 | — | 2,7 |
| | | | | | 38,5 | PHOBL | | | |

Typ = Charakterisierung der NCO-Prepolymeren (PP)
O   = Hydrophob
B   = Basisch mit tert. Stickstoff, zur Kationenbildung befähigt, enthält 246 Milliäquivalente tert.-Stickstoff/kg BOPP
K   = Kationisches NCO-Prepolymer, enthält 202 Milliäquivalente Kationen/kg KOPP
(Mengenangaben in Gewichtsteilen)

Eingesetzte Isocyanate:
    TDI = Toluylendiisocyanat-2,4-, -2,6-Isomerengemisch 80:20

Polyetherpolyole:
    PHILV = hydrophile, verzweigter Polyether, gestartet auf Trimethylolpropan, umgestzt mit 40 Teilen Propylenoxid und 60 Teilen Ethylenoxid, OH-Zahl 26.
    PHOBV = hydrophober, verzweigter Polyether, gestartet auf Trimethylolpropan mit 80 Teilen Propylenoxid und danach 20 Teilen Ethylenoxid umgesetzt, OH-Zahl 28.
    PHOBL = hydrophober, linearer Polyether aus 1,4-Butandiol und Propylenoxid, OH-Zahl 56.

Verbindung mit tertiärem Stickstoff:
    NM = N-Methyl-diethanolamin

Stabilisierungsmittel (partielle Salzbildung):
    SS = konzentrierte Schwefelsäure

Quarternierungsmittel:
    DMS = Dimethylsulfat

16

2.2 Herstellung wäßriger Polyurethanharnstoff-Dispersionen (PUR(HS)) aus NCO-Prepolymeren

2.2.1. Kationische PUR-Dispersion 1 = KPUR1

Die entwässerte Mischung der linearen und verzweigten hydrophoben Polyetherpolyole, Zusammensetzung siehe Tabelle 1, wird ca. 100 bis 130°C heiß mit dem auf Raumtemperatur belassenen Toluylendiisocyanat-Isomerengemische in einem Durchflußmischer 1, d.h. einem mit Stacheln im Stator und Rotor ausgerüstetem Stachelrührermischer mit hoher Drehzahl, vereinigt und nach einer mittleren Verweilzeit von ca. 20—60 Sekunden ein NCO-Prepolymer gebildet, das in einem dahintergeschalteten Durchflußmischer 2, gleiche Art wie 1, mit der 2,1-fachen Menge, bezogen auf NCO-Prepolymer, 20—30°C warmem Wasser vereinigt wird. Die im Wasser in der Wärme auch ohne Katalysatoren schnell erfolgende Verlängerungsreaktion (unter Polyharnstoffbildung und $CO_2$-Abspaltung wird in einem großvolumigen Ausrührgefäß bei hoher Turbulenz vollendet. Im Falle der Herstellung der 35-%igen kationischen PUR(HS)-Dispersion KPUR1 aus dem verzweigten kationischen Polyether-NCO-Prepolymeren KOPP erfolgt die Wasserverlängerung bis zur vollständigen Reaktion der Isocyanatgruppen bei 50—70°C innerhalb 5 Minuten, bei Raumtemperatur innerhalb 15 Minuten.

2.2.2. Anionische PUR-Dispersion = APUR2

Bei der Herstellung der anionischen PUR-Dispersion APUR2 aus dem nichtionischen verzweigten Polyether-NCO-Prepolymer OPP wird anstelle von reinem Wasser wie nach 2.1.1. eine 30°C warme verdünnte wäßrige Lösung des Natriumsalzes der N-(2-Aminoethyl)-ethansulfonsäure verwendet. Die Verlängerungsreaktion mit dem Diamin ist sofort und mit dem Wasser bei 50—60°C innerhalb von etwa 30 Minuten abgeschlosssen.

Beide nach 2.2 hergestellten, wäßrigen ionischen Polyurethan-Harnstoff-Dispersionen haben einen Feststoffgehalt von 35 Gew.-% und eine Teilchengröße von 0,5—3 µm, neigen zu teilweiser Sedimentation beim Stehen innerhalb mehrerer Tage, sind aber nach beliebiger Lagerung redispergierbar und als Binde- und Umhüllungsmittel zur Herstellung der erfindungsgemäßen hochgefüllten Polymerträgermassen besonders gut geeignet, wenn eine Langzeit-Wasserbeständigkeit der Träger gefordert wird.

3. Charakteristik der ionischen PUR-Dispersionen

KPUR1: Kationische, vernetzte Polyether-PUR(HS)-Dispersion aus dem NCO-Prepolymer (KOPP (siehe Tabelle 1) und Wasser, 35%ig.

APUR2: Anionische, vernetzte Polyether-PUR(HS)-Dispersion aus dem NCO-Prepolymer OPP (Tabelle 1), einem Diolsulfonat und Wasser, 35%ig.

APUR3: Anionische, vernetzte Polyether-PUR(HS)-Dispersion aus einem verzweigten und linearen Polyether (Gewichtsverhältnis 88:12), n-Methylpyrrolidon, Dimethylolpropionsäure, Triethylamin und Isophorondiisocyanat.

KPUR4: Kationische, schwach vernetzte PUR(HS)-Dispersion aus einem Gemisch aus einem linearen Polyether- und Polycarbonatdiolen im Gewichtsverhältnis 35:65, Trimethylolpropan, N-Methyldiethanolamin und Dimethylsulfat, sowie Hexamethylendiisocyanat.

4. Charakteristik von Polymerisat-Dispersionen

Die Dispersionen werden als Latex, abgekürzt LAT bezeichnet; A = anionisch, K = kationisch, NLAT = Naturkautschuklatex

KLAT1: Kationisches Polymerisat, 40%ige Dispersion aus Butadien, Acrylnitril und Trimethylolammoniumethyl-methacrylsäureethylesterchorid im Gewichtsverhältnis 68:28:4.

ALAT2: Anionisches Polymerisat, 40%ige Dispersion aus Butadien, Acrylnitril und Natrium-Methacrylat im Gewichtsverhältnis 52:41:7; 40%ig, vor SHORE A-Härte de Films: 50.

ALAT3: Anionische, 40%ige Dispersion analog ALAT2, aber deutlich weichere Einstellung (SHORE A:20) aus Butadien, Acrylnitril und Natriummmethacrylat im Gewichtsverhältnis 62:34:4.

ALAT4: Anionische Polymerisat, 50%ige Dispersion aus Styrol, Acrylnitril und Natriummmethacrylat im Gewichtsverhältnis 55:42:3.

ALAT5: Anionisches Polymerisat, 40%ige Dispersion aus Styrol, Butadien und Natrium-Acrylat im Gewichtsverhältnis 78:20:2.

ALAT6: Anionisches Polymerisat, 40%ige Dispersion aus gleichen Gewichtsteilen Acrylsäurebutylester und Vinylacetat mit 2 Gew.-% Natrium-Acrylat, bezogen auf Feststoff.

KLAT7: Kationisches Polymerisat mit untergeordnetem Anionengehalt, d.h. partiell ampholytische 40%ige, wässrige, wärmesensible Dispersion aus 2-Chlorbutadien (Chloropren).

NLAT8: Naturkautschuk-Latex mit natürlichem Eiweiß stabilisiert.

5. Charakteristik des als Füllstoff verwendeten Polyether-PUR-Schaumstoffes (Beispiele 1—15)

Bei dem Weichschaumstoff wurden Mischungen von unregelmäßig zerkleinerten Abfällen verschiedener Raumgewichte (von ca. 15 bis ca. 110 kg/m$^3$) aus der großtechnischen Polyether-Polyurethan-Block- und Formschaumstoff-Produktion verwendet.

Das Trockenschüttgewicht des überwiegend aus Blockschaumstoff-Abfall bestehenden Weichschaums beträgt etwa 14 g/l. Teilchengröße von 1 mm bis 12 mm. Schüttgewichte nach Suspendierung in Wasser S1: 263 g/l; S2: 101 g/l; S3: 14 g/l; TS-S (Gehalt an Trockensubstanz in Wassersuspension): 12,5 g/l Suspension.

6.1. Allgemeine Verfahrensvorschrift zur erfindungsgemäßen diskontinuierlichen Herstellung von gefüllten Polymerträgermassen.

6.1.1. Herstellungsmethode D1 mit vorgefertigten PUR-Schaumstoffen.

Die Herstellung der in den Patentbeispielen verwendeten, hochgefüllten Polymermassen erfolgt etwa bei Raumtemperatur oder bei mäßig erhöhter Temperatur (bis 60°C) in diskontinuierlicher Weise entweder in einem Intensivmischer, der aus einem, beheizbaren, zylindrischen Behälter besteht, der auf einem drehbaren Teller schräg befestigt ist und mit einem exzentrisch einbringbaren Rührwerk ausgestattet ist, das in entgegengesetzter Richtung zur Tellerdrehung läuft, ode man verwendet für die Herstellung größere Mengen horizontal montierte Mischer, die mit pflugscharähnlichen Werkzeugen ausgerüstet sind.

Die Füllstoffe werden vorgelegt, gegebenenfalls die über den Wassergehalt der Polymer-Dispersion hinausgehende Menge Wasser hinzugefügt und die wäßrige Polymerdispersion intensiv eingerührt. Die Mischung wird innerhalb von etwa 2—3 Minuten, gegebenenfalls mit einer verdünnten wäßrigen Lösung, Emulsion oder Suspension des vorausgewählten Koagulanz (Elektrolyt oder Polyelektrolyt) versetzt und gegebenenfalls erwärmt, wobei die Mischleistung stark gedrosselt werden muß, um unerwünschten Feinkornanteil zu vermeiden.

6.1.2. Herstellungsmethode D2 ohne vorgefertigte PUR-Schaumstoffe.

Bei Verzicht auf vorgefertigte PUR-Schaumstoff-Granulate wird nur 30—60 Sekunden bei Raumtemperatur oder ca. 30 Sekunden bei 45—60°C intensiv gemischt und dann gegebenenfalls das Koagulanz während eines Zeitraums von ca. 10 Sekunden eingerührt. Danach läßt man das Mischgut stehen, bis die Koagulation beendet ist. Das körnige Produkt wird anschließend auf emaillierte Bleche aufgetragen und getrocknet. Je nach Mischwerkzeug, angewendeter Menge Wasser und Koagulationsgeschwindigkeit und auch in Abhängigkeit von der Temperatur entsteht schon in der letzten Phase des Rührens ein mehr oder weniger körniges bis kugeliges Koagulat, da die später gewünschte Teilchengröße aufweist. Gröbere Anteile können nach der vollständigen Koagulation in einem Zerhacker auf die erforderliche Teilchengröße zerkleinert werden. In Fällen der Verwendung von vorgefertigten Schaumstoffen kann das Reaktionsgut auch während der Koagulation wenigstens noch mäßig im Mischbehälter bewegt werden, wobei entsprechend der Teilchengröße des eigesetzten Schaumstoffs hochgefüllte Polymerträgerteilchen entstehen.

6.1.3. Herstellungsmethode D3 (Verbundschaumstoff)

In einer speziellen Ausführungsform werden die vorgefertigten Schaumstoffgranulate enthaltenden Verfahrensprodukte nach Einmischung aller Komponenten innerhalb insgesamt 40—120 Sekunden in stabile Formen gebracht und je nach gewünschtem Raumgewicht auf das entsprechende (im Vergleich zum Schüttgewicht der Mischungen A—D etwa 5—12 Volumenprozent betragende) Volumen zusammengepreßt und koaguliert. Dabei entsteht ein Verbundschaumstoff, der in beliebige Formen geschnitten oder granuliert werden kann. Bei dieser Methode erhält man Polymer-gebundene Verfahrensprodukte, die ein vergleichsweise vielfach höheres Raumgewicht aufweisen, als die drucklos koagulierten Produkte. Für Patentbeispiel 13 wurde ein Raumgewicht von 280 kg/m$^3$, für Patentbeispiel 14 ein Raumgewicht von 160 kg/m$^3$ eingestellt. Rezepturen und physikalische Eigenschaften der auf unter 6 mm granulierten Verbundschäume werden in Tabellen 2 und 3 mitgeteilt.

6.2. Allgemeine Verfahrensvorschrift zur kontinuierlichen, erfindungsgemäßen Herstellung von Polymerträgermassen (kontinuierliches Verfahren = KV)

Als Apparatur dient ein Doppelpaddel-Schneckentrog mit einem Fassungsvolumen von ca. 180 l und einer Länge von ca. 300 cm, dessen Paddelwellen sich gegenläufig drehen. Die Produktförderung erfolgt zwangsweise von der eintragsöffnung in Richtung Austragsöffnung, wobei zwischen den Paddelwellen eine gewisse Knetung bzw. Quetschung des Reaktionsgemisches erfolgt. Der zerkleinerte Polyurethanschaumstoff-Abfall und weitere Füllstoffe werden getrennt über Dosierschnecken in den Schneckentrog gefördert. An gleicher Stelle wird mittels Kolbenpumpen das gegebenenfalls zusätzlich erforderliche Wasser bzw. der wäßrige Polymerisat-Latex und gegebenenfalls niedermolekulare Polyisocyanate, bzw. mittels Zahnradpumpen das gegebenenfalls mitverwendete NCO-Prepolymer eingetragen. Es ist zweckmäßig, aber nicht unbedingt erforderlich, die Polyisocyanatverbindungen mit der Polymerdispersion oder einer etwa 2-fachen Menge Wasser mit einer Temperatur von 10—25°C in einem Durchflußmischer oder statischen Mischer innerhalb von 1—3 Sekunden intensiv zu vermischen und so in eine Emulsion überzuführen, weil dadurch die gegebenenfalls mit verwendeten, getrocknete zusätzlichen Füllstoffe außerordentlich schnell und gleichmäßig mit der evtl. getrennt zudosierten Restmenge des bevorzugt auf 30—60°C erhitzten Wassers benetzt wird und die wäßrigen Polymeren in feinstverteilter Form die Feststoffe gleichmäßig umhüllen.

Nach einer Verweilzeit im Schneckentrog von etwa 3 Minuten wird im letzten Drittel des Mischaggregats eine verdünnte wäßrige Lösung des Koagulanz mit Hilfe einer Düse (Durchmesser: 1 mm) eingesprüht. Durch eine am Ende des Troges unten befindliche Offnung wird die vorgelierte und teilweise schon koagulierte Polymerträgermasse mit Hilfe eines Fließbandes in einen Heißluft-Trockenkanal geleitet und die Koagulation innerhalb von 3—10 Minuten bei einer Produkttemperatur von 40—90°C vervollständigt. In einem nachgeschalteten, geneigten Drehrohrwäscher mit Zwangsförderung von oben nach unten, in dem sich koaxial im Inneren ein Rohr Lochbleck befindet in dem Waschdüsen installiert sind, wird mit soviel Wasser gewaschen, daß sowohl eventuell störendes Feinstgut oder lösliche Salze beim Austragen des Reaktionsgutes eliminiert sind. Bei einer durchschnittlichen Verweilzeit von 2 Minuten wird etwa die 3-fache Menge Wasser, bezogen auf den Trockensubstanzgehalt der Polymerträgermasse, zum Waschen benötigt. Pro Stunde wird ein Durchsatz von etwa 1—2 Tonnen, bezogen auf Gesamtrezeptur, erreicht.

6. Patentbeispiele 1—24
(Herstellung und Verwendung der erfindungsgemäßen Polymerträgermassen)

Beispiel 1 (Herstellung)

Poröse, weichelastische, mit KLAT1 gebundene, PUR-Schaumstoff und Braunkohle enthaltende Polymerträgermasse.

40 Gew.-Teile des unter 5, beschriebenen Weichschaumgranulats und 53,8 G$\frac{1}{2}$ew.-Teile einer nativen thermisch stark entwässerten Braunkohle aus dem Aachener Braunkohlerevier mit 7 Gew.-% Restfeuchte, welche zu Teilchen unter 100 µm zerkleinert wurde, und somit als Braunkohlenstaub vorliegt, werden in einem Eirichmischer nach Methode D1 bei Raumtemperatur vorgelegt und mit 25 Gew.-Teilen des Latex KLAT1 durch intensives Rühren vereinigt. Nach 2 Minuten werden 10 Gewichtsteile einer 2%igen Schwefelsäurelösung zugefügt und die Rührwirkung stark gedrosselt und das Reaktionsgut auf 90°C erwärmt. Es entsteht innerhalb 10 Minuten ein Trägermaterial in Form eines wassergequollenen, leicht elastischen Feststoffes, der weitgehend in Stücken mit einer Größe unter 12 mm verbleibt und im allgemeinen nicht weiter zerkleinert werden muß.

Das erhaltene, in Wasser nicht ausblutende Trägermaterial wird nun in überschüssigem Wasser suspendiert, 24 Stunden (bei Raumtemperatur) vollständig gequollen und gegebenenfalls das darüber stehende Wasser abdekantiert. Der hieraus abgeleitete Wert, der die Gewichtsprozentmenge Wassers im und zwischen dem gequollenen Träger (füllstoffhaltigem Polyurethanharnstoff) angibt, wird hier als Wasseraufnahmefähigkeit (WAF) bezeichnet.

Im Beispiel 1 beträgt beispielsweise der Trockensubstanzgehalt von 1 Liter der Suspension ohne überstehendes Wasser 59,5 g Feststoff. Bei einem Suspensionsgewicht von 1010 g pro Liter Suspension errechnet sich hieraus der Suspensionsfaktor

$$F4 = \frac{1010}{59,5} = 16,8,$$

Ein Gewichtsteil Trockensubstanz der Trägermasse wird somit der 15,8-fachen Wassermenge in die beschriebene gequollene Suspensionsform überführt. Anders ausgedrückt, beträgt der Wert der Wasseraufnahmefähigkeit 15,8 durch 16,8 mal 100 = 94%.

Zur weiteren Charakterisierung der Trägermassen werden noch die Schüttgewichte S1 bis S3 (in g/l) nach unterschiedlichen Behandlungsarten bestimmt.

Im oben angeführten Beispiel betragen die so bestimmten Werte von S1 bis S3:

S1 (abgetropft) 492 g/l

S2 (ausgequetscht) 214 g/l

S3 (getrocknet) 73,5 g/l.

Die Zusammensetzung und die Werte der Volumen-, Quetsch- und Quellfaktoren F1 bis F3 von Beispiel 1 werden in den nachfolgenden Tabellen 2 und 3 zusammen mikt den entsprechenden Daten weiterer Beispiele aufgeführt.

# EP 0 209 790 B1

TABELLE 2

Zusammensetzung und Herstellungsmethode der hochgefüllten Polymerträgermassen für die Beispiele 1—15 mit Polyether-PUR-Weichschaumstoff-Abfall, Flockengröße 1—12 mm; Patentbeispiele 16—20 ohne PUR-Weichschaumstoff.

| Bsp. | PSF | BK | AK | BKK | MAG | PUR | LAT | NCO | EL | HM | WS | MIRF | TS(S) | S1 | S2 | S3 |
|------|-----|-----|-----|-----|-----|------|------|-------|-----|-----|-----|-------|-------|-----|-----|------|
| 1 | 2 | 3 | 4 | 5 | 6 | 7 | 8 | 9 | 10 | 11 | 12 | 13 | 14 | 15 | 16 | 17 |
| 1 | 40 | 50 | — | — | — | — | 10K1 | — | Mg | D1 | 90 | 50/10 | 59,5 | 492 | 214 | 73,5 |
| 2 | 50 | — | 33 | — | — | — | 15A2 | 2TD1 | — | D1 | 80 | 50/40 | 40,3 | 335 | 183 | 48 |
| 3 | 40 | 50 | — | — | — | — | 8A3 | — | S1 | D1 | 90 | 50/35 | 59,5 | 427 | 196 | 73,5 |
|   |    |    |    |    |    |    | 4A4 |    |    |    |    |       |       |     |     |      |
| 4 | 40 | 50 | — | — | — | — | 10A6 | — | — | D1 | 95 | 50/20 | 48,6 | 412 | 315 | 57,8 |
| 5 | 40 | — | — | 50 | — | — | 10N8 | — | S2 | D1 | 60 | 50/40 | 76,4 | 529 | 235 | 89,7 |
| 6 | 35 | 50 | — | — | — | — | 15A3 | — | Mg | KV | 85 | 50/25 | 59,6 | 430 | 233 | 69 |
| 7 | 40 | 50 | — | — | — | 10K4 | — | — | Mg | D1 | 90 | 50/35 | 53,8 | 457 | 234 | 58,9 |
| 8 | 50 | 10 | — | — | 30 | 10K1 | — | — | Mg | D1 | 80 | 50/40 | 51,8 | 387 | 155 | 56 |
| 9 | 50 | — | — | 8 | 30 | 10A2 | — | 20PP | — | D1 | 85 | 50/35 | 54,23 | 467 | 180 | 56,7 |
| 10 | 40 | 45 | — | — | — | 15A3 | — | — | S1 | KV | 90 | 50/40 | 61,5 | 500 | 201 | 69,5 |
| 11 | 36 | 50 | — | — | — | 7K1 | 7A3 | — | — | D1 | 60 | 50/45 | 63,2 | 467 | 194 | 63,2 |
| 12 | 40 | 50 | — | — | — | 5K1 | 5K7 |     | — | D1 | 80 | 50/35 | 60,0 | 365 | 197 | 68 |
| 13 | 60 | 20 | — | — | — | — | 20K1 |     | Mg | D3 | 90 | 50/30 | 61 | 474 | 223 | 58 |
| 14 | 88 | — | — | — | — | — | 8A2 | 40PP | Mg | D3 | 90 | 50/40 | 43,5 | 391 | 158 | 39,4 |
| 15 | 30 | — | — | 50 | | 20K1 | — | — | — | D1 | 95 | 50/35 | 99,5 | 483 | 289 | 107 |
| 16 | — | 70 | — | — | — | 30K1 | — | — | — | D3 | 90 | 35/12 | 291 | 644 | 644 | 373 |
| 17 | — | — | — | 70 | — | — | 30K1 | — | — | D3 | 95 | 33/25 | 291 | 479 | 479 | 333 |
| 18 | — | 65 | — | — | — | — | 35A2 | — | Mg | D3 | 85 | 35/25 | 173 | 681 | 664 | 216 |
| 19 | — | — | 60 | — | — | 40K4 | — | — | Mg | D3 | 90 | 37/20 | 186 | 573 | 573 | 186 |
| 20 | — | — | — | 70 | — | — | 25K1 | 5BOPP | S1 | D3 | 85 | 35/18 | 250 | 488 | 488 | 307 |

Erläuterungen zu Tabelle 2

Mengenangaben in Gewichtsteilen, bezogen auf Trockensubstanz

Spalten

| | |
|---|---|
| 1 | laufende Nummer der Patentbeispiele |
| 2—13 | Zusammensetzung und Reaktionsbedingungen |
| 2—6 | Füllstoffe A |
| 2 | PSF = Polyether-PUR-Schaumstoff-Abfall, Charakteristik siehe unter 5. |
| 3 | Braunkohlenstaub, siehe Beispiel 1 |
| 4 | Aktivkohle < 10 µm, 50 Gew.-% < µm |
| 5 | Braunkohlenkoksstaub < 200 µm, 80 Gew.-% < 100 µm |
| 6 | Magnetit, Teilchengröße 0,1—2 µm |
| 7+8 | Polymerdispersionen |
| 7 | Kationische (K) bzw. anionische (A) PUR-Dispersion, siehe Punkt 3 |
| 8 | Kationische (K) bzw. anionische (A) Polymerisatdispersionen, siehe Punkt 4 |
| 9 | NCO-Verbindungen |
| | TDI = 2,4- und 2,6-Diisocyanatotoluol im Gewichtsverhältnis 80:20 |
| | OPP = hydrophobes NCO-Prepolymer, siehe Tabelle 1 |
| 10 | Elektrolytlösungen |
| | Mg = 2%ige Magnesiumsulfatlösung (ca. 0,2 Gew.-% Magnesiumsulfat, bezogen auf Polymerfeststoff) |
| | S1 = 1n Schwefelsäurelösung (ca. 0,2 Gew.-% $H_2SO_4$, bezogen auf Polymerfeststoff) |
| | S2 = 5%ige Essigsäurelösung (ca. 0,4 Gew.-% Eisessig, bezogen auf Polymerfeststoff) |
| | L = 1n Natriumhydroxidlösung (ca. 0,3 Gew.-% Natriumhydroxid, bezogen auf Polymerfeststoff) |
| 11 | Herstellungsmethoden siehe Beschreibung Punkt 6 (D = diskontinuierlich, KV = kontinuierlich) |
| 12 | Wärmesensibilisierung, Temperatur in °C (Koagulation) |
| 13 | Wassergehalt (Gew.-%), bezogen auf Gesamtmenge |
| | M = Wassergehalt der Mischung vor de Koagulation |
| | RF = Restfeuchte nacht der Koagulation und gegebenenfalls partieller Trocknung |
| 14—17 | Physikalische Eigenschaften |
| 14 | Trockensubstanzgehalt (kg pro m³ Suspension bei 100% Füllung, d.h. ohne überstehendes Wasser) |
| 15—17 | $S_1$—$S_3$, Schüttgewichte, siehe Beispiel 1 (abgetropft, abgequetscht bzw. getrocknet) |

TABELLE 3

Volumen- (F1), Quetsch- (F2) und Quellfaktoren (F3) der hochgefüllten Polymerträgermassen für die Beispiele 1—20, ferner Suspensionsfaktoren (F4), Wasseraufnahme (WAF) und Feststoffgehalt der Suspensionen (FKS).

| Beispiel | F1 | F2 | F3 | F4 | %WAF | %FKS |
|---|---|---|---|---|---|---|
| 1 | 8,3 | 3,6 | 4,7 | 16,8 | 94,0 | 5,0 |
| 2 | 8,3 | 4,5 | 5,4 | 24,8 | 96,0 | 4,0 |
| 3 | 7,2 | 3,3 | 5,1 | 16,8 | 94,0 | 6,0 |
| 4 | 8,5 | 6,5 | 5,0 | 20,6 | 95,1 | 4,9 |
| 5 | 6,9 | 3,1 | 4,4 | 13,2 | 92,4 | 7,6 |
| 6 | 7,2 | 3,9 | 5,3 | 16,8 | 94,0 | 6,0 |
| 7 | 8,5 | 4,4 | 5,2 | 18,6 | 94,6 | 5,4 |
| 8 | 7,5 | 3,0 | 4,6 | 19,3 | 94,8 | 5,2 |
| 9 | 8,6 | 3,3 | 5,2 | 18,5 | 94,6 | 5,4 |
| 10 | 8,1 | 3,3 | 5,2 | 16,3 | 93,9 | 6,1 |
| 11 | 7,4 | 3,1 | 4,7 | 15,9 | 93,7 | 6,3 |
| 12 | 6,1 | 3,3 | 4,1 | 16,7 | 94,0 | 6,0 |
| 13 | 7,8 | 3,7 | 4,9 | 16,5 | 93,9 | 6,1 |
| 14 | 9,0 | 3,6 | 5,7 | 23,1 | 95,7 | 4,3 |
| 15 | 4,9 | 2,9 | 3,3 | 10,1 | 90,0 | 10,0 |
| 16 | 2,2 | 2,2 | 2,8 | 3,5 | 70,9 | 29,1 |
| 17 | 1,7 | 1,7 | 1,7 | 3,5 | 71,4 | 28,6 |
| 18 | 3,9 | 3,8 | 3,5 | 5,8 | 82,8 | 17,2 |
| 19 | 3,1 | 3,1 | 3,3 | 5,4 | 81,5 | 18,5 |
| 20 | 2,0 | 2,0 | 2,0 | 4,0 | 75,0 | 25,0 |

Patentbeispiele 21—24

Verwendung von Trägermassen im biologischen Reinigungsverfahren (erfindungsgemäß) Charakterisierung des angewendeten biologischen Festbett-/Wirbelbett-Bioreaktors.

Hierbei wird das aerobe Verfahren nach der Festbett-Methode als Verfahren Ia) bezeichnet, das Verfahren nach der Wirbelbett-Methode entsprechend als Verfahren Ib).

Ein Teilstrom des Ablaufs einer ersten Belebungsstufe einer industriellen Großanlage mit CSB-Werten von 350 ± 100 mg/l gelegentlich + 250 mg/l, und $BSB_5$-Werten von 23 ± 15 mg/l wird kontinuierlich in einen turmartigen Bioreaktor mit 100 l Fassungsvolumen gepumpt. Der Bioreaktor wird bis zu 2/3 mit dem Trägermaterial gefüllt, auf dem sich die Belebtschlammmase ansiedeln soll, d.h. die Polymerträgermasse nimmt eine Volumen von 66,6% ein. Das Zur Begasung und zur Sauerstoffversorgung des Reaktors notwendige Gas wird mit einer Fritte oder Lochplatte von unten dem Reaktor zugeführt. Durch die Zufuhr großer Mengen an sauerstoffhaltigem Gas kann die Säule als Wirbelbett betrieben werden oder bei geringer Gaszufuhr als Festbett. Das sauerstoffhaltige Gas tritt an der Fritte oder Lochplatte in Form kleiner Bläschen aus und durchströmt den Reaktor von unten nach oben zusammen mit dem ebenfalls unten zugeführten Wasser und tritt über den oberen Teil des Reaktors aus. Aus der Füllung (Polymerträgermasse) des Reaktors bildet sich innerhalb weniger Tage ein biologischer Rasen. Das behandelte Abwasser wird nach einer durchschnittlichen Verweilzeit von 4 Stunden über den Ablauf in eionen Klärer eingeleitet. Aus dem Bioreaktor herausgespülte Teilchen des biologischen Rasens scheiden sich im Klärer ab und können über einen Absperrhahn abgezogen werden. Das biologisch gereinigte Abwasser verläßt den Klärer mit den in den Beispielen angegebenen verbesserten Ergebnissen.

Die Anlaufzeit liegt bei 4 Wochen. Die in Tabelle 4 mitgeteilten Analysendaten stellen Durchschnittswerte von jeweils 5 Analysen dar. Bei der anaeroben biologischen Reinigung wird im wesentlichen nach de Fetbett-Methode verfahren und lediglich zur Vermeidung von Konzentrationsunterschieden im Bioreaktor gelegentlich anstelle von Luft ein inertes Trägergas in Intervallen eingeleitet oder für eine periodische, schwache, mechanische Bewegung des Trägermaterials gesorgt.

Patentbeispiele 21—24

TABELLE 4

(CSB-Eliminisierung bei der aeroben, biologischen Reinigung, CSB = chemischer Sauerstoffbedarf)

| Bsp. | Trägermasse gemäß Bsp. | Zulauf | Ablauf | Verfahren | CSB-Eliminiereung mg/l | % |
|---|---|---|---|---|---|---|
| — | ohne | 379 | 357 | — | 22 | 6 |
| | " | 240 | 211 | — | 29 | 12 |
| | " | 682 | 660 | — | 20 | 3 |
| 21 | 1 | 379 | 170 | la | 209 | 55 |
| 22 | 6 | 379 | 141 | lb | 238 | 63 |
| 23 | 15 | 240 | 129 | la | 111 | 45 |
| 24 | 1 | 682 | 218 | lb | 462 | 68 |

Beispiele 25 und 26

Abwässer aus der Sauerstoffbleiche einer Sulfitzellstoffabrik, deren Gehalt an CSB 5500 mg/l betrug, wurden in parallel-kontinuierlich betriebenen Anlagen einer anaeroben mikrobiellen Behandlung unterzogen.

Die Versuche wurden in 1,6 l fassenden Anaerobanlagen, wie sie z.B. von W. J. Jewell in "Journal of the Water Pollution Control Federation", Vol. 53, Nr. 4, S. 484, Abb, 1b beschrieben sind, durchgeführt. Die mittlere hydraulische Veweilzeit des Abwassers im Reaktor betrug 38 Std. (1,6 Tage). Die Abauversuche wurden in folgenden Varianten durchgeführt:

Anlage 1 (Nulltest ohne Trägerzusatz — als Vergleich) (Beispiel 25)
400 ml suspendierte Zellen

Anlage 2 (erfindungsgemäß)
400 ml suspendierte Zellen plus 400 ml PUR-Polymerträgermassen von Patentbeispiel 1 (siehe Tabelle 2, S. 68)

Anlage 3 (Biespiel 26)
400 ml suspendierte Zellen und 400 ml PUR-Polymerträgermassen nach Beispiel 6, Tabelle 2, S. 68.

Die suspendierten Zellen wurden dem Anaerob-Reakator einer Zuckerfabrik entnommen.
Nach Erreichen der Gleichgewichtsbedingungen (34 Tage) wurden folgende Ergebnisse erzielt:

| | Anlage Nr. | CSB im Ablauf (mg/l) | Polymerträgermasse nach |
|---|---|---|---|
| Nulltest | 1 | 2190 | — |
| Beispiel 25 | 2 | 1630 | nach Beispiel 1, Tabelle 2 |
| Beispiel 26 | 3 | 1670 | nach Beispiel 6, Tabelle 2 |

**Patentansprüche**

1. Verfahren zur Herstellung von Füllsoffe enthaltenden, Polymer-gebundenen Trägermassen, dadurch gekennzeichnet, daß man

(A) Füllstoffe auf Basis von vorgefertigten, stückigen oder pulverförmigen Schaumstoffen und/oder von fossilen Lignozellulosepulvern und/oder von Kohlenpulvern, sowie gegebenenfalls weiteren anorganischen oder organischen Füllstoffen und/oder gegebenenfals von lebendem und/oder totem biologischem Zellmaterial in einer Menge, bezogen auf A) und B) von 5 bis 97 Gew.-%, mit

E) nichtionisch-hydrophilen, anionischen oder kationischen wäßrigen, koagulierbren und filmbildenden Polymerdispersionen mit einem Feststoffgehalt von 3 bis 60 Gew.-%, gegebenenfalls unter Zusatz von

C) Wasser, so daß der Gesamtwassergehalt, bezogen auf alle Bestandteile, 20 is 90.-% beträgt, und gegebenenfalls unter Zusatz von

D) weiteren Hilfs- und Zusatzmitteln vermischt und anschließend die Polymerdispersion zur Koagulation bringt.

2. Verfahren gemäß Anspruch 1, dadurch gekennzeichnet, daß man als Komponente A) Füllstoffe ausgewählt aus der Gruppe bestehend aus

a) stückigen oder pulverförmigen Schaumstoffen,

b) fossilen Lignozellulosepulvern, vorzugsweise Braunkohlenpulvern oder Torf,

c) anderen Kohlepulvern, vorzugsweise Aktivkohlen-, Steinkohlen-, Braunkohlenkoks- oder Steinkohlenpulvern und

d) beliebigen Gemischen derartiger Füllstoffe, gegebenenfalls in Kombination mit

e) pulverförmigen, ferromagnetischen anorganischen Stoffen verwendet.

3. Verfahren gemäß Ansprüchen 1 und 2, dadurch gekennzeichnet, daß man als Komponente A) Füllstoffe ausgewählt aus der Gruppe bestehend aus

a) stückigen oder pulverförmigen Schaumstoffen,

b) und/oder fossilen Lignozellulosepulvern, vorzugsweise Braunkohlenpulvern oder Torf,

c) gegebenenfalls in Gegenwart anderer Kohlepulver, vorzugsweise Aktivkohlen-, Steinkohlen-, Braunkohlenkoks- oder Steinkohlenpulver und

d) beliebigen Gemischen derartiger Füllstoffe, gegebenenfalls in Kombination mit

e) pulverförmigen, ferromagnetischen anorganischen Stoffen verwendet.

4. Verfahren gemäß Ansprüchen 1 bis 3, dadurch gekennzeichnet, daß man als wäßrige Polymerdispersionen solche ausgewählt aus der Gruppe bestehend aus

a) Polymerdispersionen auf Basis von olefinisch ungesättigten Monomeren,

b) Polyurethandispersionen,

c) Naturlatex und

d) beliebigen Abmischungen derartiger miteinander verträglicher Dispersionen verwendet, wobei die Stabilität der Dispersionen durch externe, chemisch nicht eingebaute und/oder durch interne, chemisch in das Makromolekül der Festkörper eingebaute nichtionisch-hydrophile, anionische und/oder kationische Emulgatoren bzw. hydrophile Gruppierungen gewährleistet ist.

5. Verfahren gemäß Ansprüchen 1 bis 4, dadurch gekennzeichnet, daß man als gegebenenfalls mitzuverwendende weitere Hilfs- und Zusatzmittel D) stabilisierend und/oder vernetzend wirkende Zusatzmittel verwendet.

6. Verfahren gemäß Ansprüchen 1 bis 5, dadurch gekennzeichnet, daß man die Koagulation durch Einwirken eines Koagulanz und/oder durch Hitzeinwirkung bewirkt.

7. Gemäß Ansprüchen 1 bis 6 erhaltene, Polymer-gebundene Trägermassen.

8. Verwendung der gemäß Anspruchen 1 bis 6 erhaltenen, Polymer-gebundenen Trägermassen als Träger für inkorporierte oder aufwachsende Biomassen bei der Abwasserreinigung, insbesondere bei der biologischen Abwasserreinigung, als Träger bei der biologischen Fermentation in Biokonversionsprozessen, als Träger fur Pflanzenwachstum oder als Adsorbentien für feindispergierte Stoffe und/oder Rohöl.

**Revendications**

1. Procédé de production de matières de support liées à un polymère, contenant des charges, caractérisé en ce qu'on mélange

A) des charges à base de mousses préformées, en morceaux ou en poudre et/ou de poudres lignocellulosiques fossiles et/ou de poudres de charbon, ainsi que, le cas échéant, d'autres charges inorganiques ou organiques et/ou, le cas échéant, d'un matière cellulaire biologique vivante et/ou morte, en une quantité, par rapport à A) et B), de 5 à 97% n poids, avec

B) de dispersions aqueuses, coagulables et filmogènes, non ioniques-hydrophiles, anioniques ou cationiques, de polymère, avec une teneur en matiére solide de 3 à 60% en poids,
en ajoutant le case échéant

(C) de l'eau, de manière que la teneur totale en eau, par rapport à tous les composants, s'élève à 20—90% en poids,

et le cas échéant avec addition

D) d'autres ajuvants et additifs,

puis on fait coaguler la dispersion de polymère.

2. Procédé suivant la revendication 1, caractérisé en ce qu'on choisit comme composant A) des charges choisies dans le groupe comprenant

a) des mousses en morceaux ou en poudre,

b) des poudres lignocellulosiques fossiles, de préférence des poudres de lignite ou de la tourbe,

c) d'autres poudres de charbon, de préférence des poudres de charbon actif, de houille, de coke de lignite ou de houille et

d) des mélanges quelconques de ces charges, le cas échéant en association avec

e) des matières inorganiques ferromagnétiques en poudre.

3. Procédé suivant la revendications 1 et 2, caractérisé en ce qu'on choisit comme composant A) de charges du groupe comprenant

a) de mousses en morceaux ou en poudre,

b) et/ou des poudres lignocellulosiques fossiles, de préférence des poudres de lignite ou de la tourbe,

c) le cas échéant en présence d'autres poudres de charbon, de préférence des poudres de charbon actif, de houille, de coke de lignite ou de houille et

d) des mélanges quelconques de ces charges, le cas échéant en association avec

e) des matières inorganiques ferromagnétiques en poudre.

4. Procédé suivant les revendications 1 à 3, caractérisé en ce qu'on choisit comme dispersions aqueuses de polymère des dispersions du groupe comprenant

a) de dispersions de polymère à base de monomères à non-saturation oléfinique,

b) des dispersions de polyuréthanne,

c) un latex naturel et

d) des mélanges quelconques de ces dispersions compatibles les unes avec les autres,

la stabilité des dispersions étant assurée par des émulsionnants externes, chimiquement non incorporés et/ou par des émulsionnants internes non ioniques-hydrophiles, anioniques et/ou cationiques chimiquement incorporés dans la macromolécule des corps solides, ou par des groupements hydrophiles.

5. Procédé suivant les revendications 1 à 4, caractérisé en ce qu'on utilise, comme autres substances auxiliaires et additifs D) pouvant être utilisés le cas échéant, des additifs à effet stabilisant et/ou à effet réticulant.

6. Procédé suivant les revendications 1 à 5, caractérisé en ce qu'on effectue la coagulation par l'action d'un agent coagulant et/ou par l'action de la chaleur.

7. Matières de support liées à un polymère, obtenues conformément aux revendications 1 à 6.

8. Utilisation des matières de support liées à un polymère obtenues conformément aux revendications 1 à 6 comme substrat pour des biomasses incorporées ou en développement dans l'épuration d'eaux usées, notamment dans l'épuration biologique d'eaux usées, comme substrat lors de la fermentation biologique dans des processus de bioconversion, comme substrat pour la croissance de plantes ou comme matières adsorbantes pour des substances finement dispersées et/ou du pétrole brut.

**Claims**

1. Process for the preparation of polymer-bound carrier masses containing fillers, characterised in that

A) fillers based on prefinished, lumpy or pulverulent voams and/or fossil ligno cellulose powders and/or carbon powders and optionally other inorganic or organic fillers and/or optionally living and/or dead biological cell material in a quantity of from 5 to 97% by weight, based on A) and B), are mixed with

B) nonionic-hydrophilic, anionic or cationic, aqueous, coagulable and film forming polymer dispersions having a solids content of from 3 to 60% by weight, optionally with the addition of

C) water so that the total water content, based on all components, amounts to 20 to 90% by weight, and optionally with the addition of

D) other auxiliary agents and additives and the polymer dispersion is then brought to coagulation.

2. Process according to Claim 1, characterised in that the fillers used as component (A) are selected from the group comprising

a) lumpy or pulverulent foams,

b) fossil ligno cellulose powders, preferably brown coal powders or peat,

c) other carbon powders, preferably active carbon powder, powdered hard coal, brown coal coke powder or hard coal powder and

d) any mixtures of such fillers, optionally in combination with

e) pulverulent, ferromagnetic inorganic substances.

3. Process according to Claims 1 and 2, characterised in that the fillers used as component A) are selected from the group comprising

a) lumpy or pulverulent foams,

b) and/or fossil ligno cellulose powders, preferably brown coal powders or peat,

c) optionally in the presence of other carbon powders, preferably active carbon powder, hard coal powder, brown coal coke powder or hard coal powder and

d) any mixtures of such fillers, optionally in combination with

e) pulverulent, ferromagnetic inorganic substances.

4. Process according to Claims 1 to 3, characterised in that the aqueous polymer dispersions are selected from the group comprising,

a) polymer dispersions based on olefinically unsaturated monomers,

b) polyurethane dispersions,

c) natural latex and

d) any mixtures of such dispersions which are compatible with one another,

the stability of the dispersions being ensured by nonionic-hydrophilic, anionic and/or cationic emulsifiers or hydrophilic groups which are external and chemically not built in and/or internal and chemically built into the macromolecule of the solid substances.

5. Process according to Claims 1 to 4, characterised in that the auxiliary agents and additives (D) optionally used are stabilizing and/or cross-linking additives.

6. Process according to Claims 1 to 5, characterised in that coagulation is brought about by the action of a coagulant and/or by the action of heat.

7. Polymer-bound carrier masses obtained according to Claims 1 to 6.

8. Use of the polymer-bound carrier masses obtained according to Claims 1 to 6 as carriers for biomasses which are incorporated or grow on the surface in the purification of effluent, in particular biological purification of effluent, as carriers for biological fermentation in bioconversion processes, as carriers for plant growth or as adsorbents for finely dispersed substances and/or crude oil.